# FASCICULE DE BREVET EUROPEEN

(11) **EP 1 525 325 B1**
(45) Date de publication et mention de la délivrance du brevet: **22.08.2012**
(21) Numéro de dépôt: 03755629.7
(22) Date de dépôt: 24.07.2003
(51) Int. Cl.: C12Q 1/68

(54) **METHODE D'ANALYSE D'ACIDES NUCLEIQUES ET SON UTILISATION POUR EVALUER LE DEGRE D'EDITION DE L'ARNM DU RECEPTEUR 5-HT2C DE LA SEROTONINE**
VERFAHREN ZUR ANALYSE VON NUKLEINSÄUREN UND VERWENDUNG ZUR EVALUIERUNG DES MRNA EDITING DES SEROTONINREZEPTORS 5-HT2C
NOVEL METHOD FOR ANALYZING NUCLEIC ACID AND USE THEREOF FOR EVALUATING THE DEGREE OF MRNA EDITING OF THE SEROTONIN 5-HT SB 2C /SB RECEPTOR

(30) Priorité: 26.07.2002 FR 0209524
(43) Date de publication de la demande: 27.04.2005
(73) Titulaire: MARCO POLO PHARMACEUTICALS, 75008 Paris (FR)
(72) Inventeur: MADJAR, Jean-Jacques, F-69002 Lyon (FR); BERTHOMME, Hervé, F-69500 Bron (FR)
(74) Mandataire: Faivre Petit, Frédérique
(86) Numéro de dépôt international: PCT/FR2003/002339
(87) Numéro de publication internationale: WO 2004/011594

(56) Documents cités:
- EP-A- 0 552 931
- WO-A-02/38809
- US-A- 5 578 467
- NISWENDER C.M. ET AL.: "Identification and characterization of RNA editing events within the 5-HT2C receptor" ANNALS OF THE NEW YORK ACADEMY OF SCIENCES, vol. 861, 1998, pages 38--48, XP008017462
- NGUYEN H-K ET AL: "Studies towards the design of a modified GC base pair with stability similar to that of the AT base pair" TETRAHEDRON LETTERS, ELSEVIER SCIENCE PUBLISHERS, AMSTERDAM, NL, vol. 38, no. 23, 9 juin 1997 (1997-06-09), pages 4083-4086, XP002041345 ISSN: 0040-4039
- NISWENDER COLLEEN M: "Strategies and requirements for the detection of RNA editing in G protein coupled-receptor RNA." METHODS IN ENZYMOLOGY. UNITED STATES 2002, vol. 343, 2002, pages 476-492, XP008029607 ISSN: 0076-6879
- ZHONG SHAOBIN ET AL: "Detection of apolipoprotein B mRNA editing by peptide nucleic acid mediated PCR clamping" BIOCHEMICAL AND BIOPHYSICAL RESEARCH COMMUNICATIONS, vol. 259, no. 2, 7 juin 1999 (1999-06-07), pages 311-313, XP002276504 ISSN: 0006-291X
- FUCHS MELANIE ET AL: "RNA editing in higher plant plastids: Oligoribonucleotide SSCP analysis allows the proof of base conversion directly at the RNA level" CURRENT GENETICS, vol. 39, no. 5-6, juillet 2001 (2001-07), pages 384-387, XP002276505 ISSN: 0172-8083
- IBRAHIM A F ET AL: "Differential expression of potato U1A spliceosomal protein genes: a rapid method for expression profiling of multigene families." PLANT MOLECULAR BIOLOGY. MAR 2001, vol. 45, no. 4, mars 2001 (2001-03), pages 449-460, XP002283623 ISSN: 0167-4412
- MAEKAWA MASATO ET AL: "Relative ratios of mRNA molecules encoded by genes with homologous sequences using fluorescence-based single-strand conformation polymorphism analysis" BIOCHEMICAL AND BIOPHYSICAL RESEARCH COMMUNICATIONS, vol. 223, no. 3, 1996, pages 520-525, XP002283624 ISSN: 0006-291X
- ELLISON JANE S: "Fluorescence-based mutation detection: Single-strand conformation polymorphism analysis (F-SSCP)" MOLECULAR BIOTECHNOLOGY, vol. 5, no. 1, 1996, pages 17-31, XP008031354 ISSN: 1073-6085
- CHANRION BENJAMIN ET AL: "Inverse agonist and neutral antagonist actions of antidepressants at recombinant and native 5-hydroxytryptamine2C receptors: differential modulation of cell surface expression and signal transduction.", MOLECULAR PHARMACOLOGY MAR 2008, vol. 73, no. 3, March 2008 (2008-03), pages 748-757, ISSN: 1521-0111
- DRACHEVA S ET AL: "Increased serotonin 2C receptor mRNA editing: a possible risk factor for suicide.", MOLECULAR PSYCHIATRY NOV 2008, vol. 13, no. 11, November 2008 (2008-11), pages 1001-1010, ISSN: 1476-5578
- LANFRANCO MARIA FE ET AL: "An innovative real-time PCR method to measure changes in RNA editing of the serotonin 2C receptor (5-HT(2C)R) in brain.", JOURNAL OF NEUROSCIENCE METHODS 15 MAY 2009, vol. 179, no. 2, 15 May 2009 (2009-05-15), pages 247-257, ISSN: 1872-678X
- POYAU ALAIN ET AL: "Identification and relative quantification of adenosine to inosine editing in serotonin 2c receptor mRNA by CE.", ELECTROPHORESIS AUG 2007, vol. 28, no. 16, August 2007 (2007-08), pages 2843-2852, ISSN: 0173-0835
- SEEBURG P H ET AL: "RNA editing of brain glutamate receptor channels: mechanism and physiology.", BRAIN RESEARCH. BRAIN RESEARCH REVIEWS MAY 1998, vol. 26, no. 2-3, May 1998 (1998-05), pages 217-229,
- FITZGERALD L W ET AL: "Messenger RNA editing of the human serotonin 5-HT2C receptor.", NEUROPSYCHOPHARMACOLOGY : OFFICIAL PUBLICATION OF THE AMERICAN COLLEGE OF NEUROPSYCHOPHARMACOLOGY AUG 1999, vol. 21, no. 2 Suppl, August 1999 (1999-08), pages 82S-90S, ISSN: 0893-133X

### LISTE DE SEQUENCES

<110> BIOCORTECH
<120> Nouvelle méthode d'analyse d'acide nucléique et son utilisation pour évaluer le degré d'édition de l'ARNm, notamment celui du récepteur 5-HT_{2C} de la sérotonine
<130> D20534
<150> FR 02/09 524
   <151> 2002-07-26
<160> 37
<170> PatentIn Ver. 2.1
<210> 1
   <211> 17
   <212> ADN
   <213> Séquence artificielle
<220>
   <223> Description de la séquence artificielle: ADN dérivé de l'ARNm codant pour le récepteur 5-HT_{2C} humain
<400> 1
   caatacgtaa tcctatt 17
<210> 2
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<400> 2
   cantacgtaa tcctatt 17
<210> 3
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (5)
   <223> n= i
<400> 3
   caatncgtaa tcctatt 17
<210> 4
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (10)
   <223> n= i
<400> 4
   caatacgtan tcctatt 17
<210> 5
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (15)
   <223> n= i
<400> 5
   caatacgtaa tcctntt 17
<210> 6
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (9)
   <223> n= i
<400> 6
   caatacgtna tcctatt 17
<210> 7
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (5)
   <223> n= i
<400> 7
   cantncgtaa tcctatt 17
<210> 8
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (10)
   <223> n= i
<400> 8
   cantacgtan tcctatt 17
<210> 9
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (15)
   <223> n= i
<400> 9
   cantacgtaa tcctntt 17
<210> 10
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (9)
   <223> n= i
<400> 10
   cantacgtna tcctatt 17
<210> 11
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (5)
   <223> n= i
<220>
   <221> modified_base
   <222> (10)
   <223> n= i
<400> 11
   caatncgtan tcctatt 17
<210> 12
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (5)
   <223> n= i
<220>
   <221> modified_base
   <222> (15)
   <223> n= i
<400> 12
   caatncgtaa tcctntt 17
<210> 13
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (5)
   <223> n= i
<220>
   <221> modified_base
   <222> (9)
   <223> n= i
<400> 13
   caatncgtna tcctatt 17
<210> 14
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (10)
   <223> n= i
<220>
   <221> modified_base
   <222> (15)
   <223> n= i
<400> 14
   caatacgtan tcctntt 17
<210> 15
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (9)
   <223> n= i
<220>
   <221> modified_base
   <222> (10)
   <223> n= i
<400> 15
   caatacgtnn tcctatt 17
<210> 16
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (9)
   <223> n= i
<220>
   <221> modified_base
   <222> (15)
   <223> n= i
<400> 16
   caatacgtna tcctntt 17
<210> 17
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (5)
   <223> n= i
<220>
   <221> modified_base
   <222> (10)
   <223> n= i
<400> 17
   cantncgtan tcctatt 17
<210> 18
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (5)
   <223> n= i
<220>
   <221> modified_base
   <222> (15)
   <223> n= i
<400> 18
   cantncgtaa tcctntt 17
<210> 19
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (5)
   <223> n= i
<220>
   <221> modified_base
   <222> (9)
   <223> n= i
<400> 19
   cantncgtna tcctatt 17
<210> 20
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (10)
   <223> n= i
<220>
   <221> modified_base
   <222> (15)
   <223> n= i
<400> 20
   cantacgtan tcctntt 17
<210> 21
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (9)
   <223> n= i
<220>
   <221> modified_base
   <222> (10)
   <223> n= i
<400> 21
   cantacgtnn tcctatt 17
<210> 22
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (9)
   <223> n= i
<220>
   <221> modified_base
   <222> (15)
   <223> n= i
<400> 22
   cantacgtna tcctntt 17
<210> 23
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (5)
   <223> n= i
<220>
   <221> modified_base
   <222> (10)
   <223> n= i
<220>
   <221> modified_base
   <222> (15)
   <223> n= i
<400> 23
   caatncgtan tcctntt 17
<210> 24
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (5)
   <223> n= i
<220>
   <221> modified_base
   <222> (9)
   <223> n= i
<220>
   <221> modified_base
   <222> (10)
   <223> n= i
<400> 24
   caatncgtnn tcctatt 17
<210> 25
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (5)
   <223> n= i
<220>
   <221> modified_base
   <222> (9)
   <223> n= i
<220>
   <221> modified_base
   <222> (15)
   <223> n= i
<400> 25
   caatncgtna tcctntt 17
<210> 26
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (9)
   <223> n= i
<220>
   <221> modified_base
   <222> (10)
   <223> n= i
<220>
   <221> modified_base
   <222> (15)
   <223> n= i
<400> 26
   caatacgtnn tcctntt 17
<210> 27
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (5)
   <223> n= i
<220>
   <221> modified_base
   <222> (10)
   <223> n= i
<220>
   <221> modified_base
   <222> (15)
   <223> n= i
<400> 27
   cantncgtan tcctntt 17
<210> 28
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (5)
   <223> n= i
<220>
   <221> modified_base
   <222> (9)
   <223> n= i
<220>
   <221> modified_base
   <222> (10)
   <223> n= i
<400> 28
   cantncgtnn tcctatt 17
<210> 29
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (5)
   <223> n= i
<220>
   <221> modified_base
   <222> (9)
   <223> n= i
<220>
   <221> modified_base
   <222> (15)
   <223> n= i
<400> 29
   cantncgtna tcctntt 17
<210> 30
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (9)
   <223> n= i
<220>
   <221> modified_base
   <222> (10)
   <223> n= i
<220>
   <221> modified_base
   <222> (15)
   <223> n= i
<400> 30
   cantacgtnn tcctntt 17
<210> 31
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (5)
   <223> n= i
<220>
   <221> modified_base
   <222> (9)
   <223> n= i
<220>
   <221> modified_base
   <222> (10)
   <223> n= i
<220>
   <221> modified_base
   <222> (15)
   <223> n= i
<400> 31
   caatncgtnn tcctntt 17
<210> 32
   <211> 17
   <212> ADN
   <213> Séquence artificielle
<220>
   <223> Description de la séquence artificielle: ADN dérivé de l'ARNm codant pour le récepteur 5-HT_{2C} humain
<220>
   <221> modified_base
   <222> (3)
   <223> n= i
<220>
   <221> modified_base
   <222> (5)
   <223> n= i
<220>
   <221> modified_base
   <222> (9)
   <223> n= i
<220>
   <221> modified_base
   <222> (10)
   <223> n= i
<220>
   <221> modified_base
   <222> (15)
   <223> n= i
<400> 32
   cantncgtnn tcctntt 17
<210> 33
   <211> 13
   <212> ARN
   <213> Séquence artificielle
<220>
   <223> Description de la séquence artificielle: ARN dérivé de l'ARNm codant pour le récepteur 5-HT_{2C} humain
<400> 33
   auacguaauc cua 13
<210> 34
   <211> 17
   <212> ARN
   <213> Séquence artificielle
<220>
   <223> Description de la séquence artificielle: ARN dérivé de l'ARNm codant pour le récepteur 5-HT_{2C} humain
<400> 34
   caauacguaa uccuauu 17
<210> 35
   <211> 17
   <212> ARN
   <213> Séquence artificielle
<220>
   <223> Description de la séquence artificielle: ARN dérivé de l'ARNm codant pour le récepteur 5-HT_{2C} humain
<220>
   <221> modified_base
   <222> (3)
   <223> n= i
<220>
   <221> modified_base
   <222> (5)
   <223> n= i
<220>
   <221> modified_base
   <222> (9)
   <223> n= i
<220>
   <221> modified_base
   <222> (10)
   <223> n= i
<220>
   <221> modified_base
   <222> (15)
   <223> n= i
<400> 35
   canuncgunn uccunuu 17
<210> 36
   <211> 25
   <212> ADN
   <213> Séquence artificielle
<220>
   <223> Description de la séquence artificielle: Amorce dérivée de l'ARNm du récepteur 5-HT_{2C}
<400> 36
   tgtccctagc cattgctgat atgct 25
<210> 37
   <211> 26
   <212> ADN
   <213> Séquence artificielle
<220>
   <223> Description de la séquence artificielle: Amorce dérivée de l'ARNm du récepteur 5-HT_{2C}
<400> 37
   gcaatcttca tgatggcctt agtccg 26

## Description

Une méthode d'analyse des acides nucléiques mettant en oeuvre un jeu de sondes de petite taille incluant des désoxyinosines (dI) à la place de désoxyguanosines (dG) est décrite. De tels jeux de sondes sont également décrits, ainsi que leur utilisation dans des procédés de détection et/ou de quantification d'oligonucléotides cibles présents dans des molécules d'ADN (acide désoxyribonucléique) ou d'ARN (acide ribonucléique) dans un échantillon, notamment la détermination du taux d'édition de l'ARN messager (ARNm) du récepteur 5-HT_{2c} (5-HT_{2c}-R) de la sérotonine. Une biopuce ou un réacteur en milieu liquide comprenant de tels jeux de sondes sont également décrits, ainsi que leurs utilisations, notamment pour la détection et/ou l'identification de polymorphismes génétiques ou pour la détermination du taux d'édition d'un ARNm, que ce soit celui de l'ARNm du 5-HT_{2c}-R ou de tout autre ARN susceptible d'être édité. La présente invention a également pour objet une méthode fondée sur la mise en évidence de polymorphisme de conformation de l'ADN simple brin (SSCP) permettant dans des conditions données d'analyse d'obtenir le profil et/ou le taux d'édition d'un ARNm susceptible d'être édité, ainsi qu'une méthode de diagnostic de maladies ou de susceptibilité à des maladies associées au degré d'édition d'un ARNm. La présente invention a aussi pour objet une méthode de sélection de composés capables de moduler le taux d'édition de l'ARNm, notamment celui du 5-HT_{2c}-R, ainsi que l'utilisation de tels composés pour la préparation d'une composition pharmaceutique destinée au traitement de l'humeur.

Parmi les méthodes d'analyse des acides nucléiques qui permettent de révéler une différence d'un ou de quelques nucléotides dans une séquence donnée, on peut distinguer deux grandes catégories de méthodes, celles qui sont spécifiques d'une seule séquence et celles qui permettent de détecter simultanément, par hybridation sous forme de duplex d'ADN double brin ou sous forme d'hétéroduplex ADN/ARN, plusieurs séquences différentes (jusqu'à plusieurs milliers).

Dans la première catégorie, on peut ranger - sans être limitatif tant les variantes sont nombreuses et sans les décrire car elles ont toutes été largement utilisées à des degrés divers - le séquençage de l'ADN, séquençage direct ou après amplification itérative par PCR ("polymerase chain reaction"), précédée ou non d'une réaction de transcription inverse (RT-PCR pour "reverse transcription" suivie de PCR) ; la mise en évidence d'un polymorphisme de longueur des fragments de restriction (RFLP pour "restriction fragment length polymorphism") à la suite d'une ou plusieurs mutations ponctuelles qui affectent un ou plusieurs sites de restriction et sa variante, l'AFLP ("amplification length polymorphism") ; la réaction de ligature itérative (LCR pour "ligase chain reaction") et sa variante, la réaction de détection par ligature (LDR pour "ligase détection reaction") ; l'amplification spécifique par PCR d'un allèle muté (PCR-MASA pour "PCR-mutant allele specific amplification") ; l'extension d'amorce ("primer extension") ; .... Pour toutes ces méthodes et celles qui en dérivent voir "Current Protocols in Molecular Biology", John Wiley & Sons ed., 4 vol. mis à jour par trimestre, ISBN 0-471-50338-X.

Dans la seconde catégorie, on trouve les méthodes d'analyse par hybridation en milieu liquide et celles dérivées de la méthode de Southern (ou "Southern blot") qui fut décrite initialement pour l'ADN (Southern, J. Mol. Biol., 1975, 98:503-517). Parmi ces dernières, on peut citer sans être limitatif, la méthode de Southern appliquée à l'ARN (ou "northern blot", voir Alwine et al., Proc. Natl. Acad. Sci. USA, 1977, 74:5350-5354, et Alwine et al., Methods Enzymol., 1979, 68:220-242) ; les hybridations sur support, macro- et micro-hybridations, d'acides nucléiques en collection ("macroarray" et "microarray", voir Fotin et al., Nucleic Acids Res., 1998, 26(6):1515-1521 et les articles qui y sont référencés, voir aussi Diehl et al., Nucleic Acids Res., 2001, 29(7)e38 et les articles qui y sont référencés). Ces hybridations entre molécules d'acides nucléiques qui aboutissent à la formation d'un ADN double brin ou d'un hétéroduplex de type ADN/ARN sont obtenues soit dans un réacteur en milieu liquide soit, plus généralement, sur un support qui peut être une membrane hydrophobe (nitrocellulose ou Nylon^{®} par exemple) ou une lame de verre activé pour permettre la fixation des sondes d'ADN simple brin ou double brin (qui, dans ce dernier cas, sera ensuite dénaturé pour permettre l'hybridation). Les séquences d'ADN déposées qui servent de sondes pour l'hybridation de molécules d'ADN ou d'ARN qui leur sont complémentaires, peuvent être longues et ce sont alors des séquences d'ADN génomique, généralement amplifiées par PCR ou, plus souvent, d'ADNc (ADN complémentaire) d'ARNm ; dans ce dernier cas, elles proviennent de banques d'ADNc d'origines diverses et se présentent sous forme d'ADN double brin. Les séquences d'ADN peuvent également être sous forme d'ADN simple brin ; dans ce dernier cas, il s'agira, le plus souvent, d'oligodésoxyribonucléotides longs (60 à 80 bases) ou courts (environ 20 bases ou moins). Plus les oligodésoxyribonucléotides sont courts, plus ils permettent de discriminer des séquences qui diffèrent ne fut-ce que d'un seul nucléotide. Cependant, cette discrimination n'est possible que si la température de fusion Tm (Tm pour "melting temperature") des séquences à hybrider est très voisine ou identique.

En effet, s'il est possible par les méthodes de la seconde catégorie de mettre en évidence une différence de séquence portant sur un seul nucléotide, il est nécessaire pour cela que les conditions d'hybridation (concentration en sels et température essentiellement) soient suffisamment strictes pour qu'un seul mésappariement de base empêche l'hybridation entre la sonde et l'acide nucléique cible. Lorsque ces conditions sont réunies, seule la sonde de séquence exactement complémentaire s'hybride à l'acide nucléique cible qui peut être de l'ADN ou de L'ARN simple brin de séquence recherchée. Pour cela, la sonde doit être de séquence suffisamment longue pour permettre une hybridation à la fois stable et spécifique mais, aussi, suffisamment courte pour autoriser une discrimination entre des populations d'acide nucléique (ADN ou ARN) qui présenteraient entre elles une différence de séquence d'un seul nucléotide.

Toutes choses étant égales par ailleurs, la température à laquelle est obtenue l'hybridation spécifique dépend essentiellement de la composition en bases des séquences à apparier, adénine, thymine (ou uracile), guanine et cytosine présentes sous forme de désoxyribonucléoside monophosphate dans l'ADN et de ribonucléoside monophosphate dans l'ARN (l'uracile remplaçant alors la thymine), reliés entre eux par des liaisons 3'-5'-phosphodiesters. On utilisera les abréviations dA, dT, dG et dC pour désoxyadénosine, désoxythymidine, désoxyguanosine et désoxycytidine monophosphates, respectivement, et A, U, G et C pour adénosine, uridine, guanosine et cytidine monophosphates, respectivement. Plus ces séquences sont riches en guanines et cytosines, plus la température qui permet d'obtenir une hybridation spécifique est élevée, en raison de la formation de trois liaisons hydrogènes entre ces deux bases appariées, contre deux seulement entre les adénines et thymines (ou uraciles) appariées. Cependant, la position relative de ces bases à l'intérieur des séquences appariées joue aussi un rôle dans la stabilité de la double hélice d'ADN ou de l'hétéroduplex ADN/ARN, en raison des interactions hydrophobes que contractent entre eux certains atomes de carbone des plateaux des bases puriques et pyrimidiques empilées. Par conséquent, du pourcentage en adénines et thymines (ou uraciles), ainsi qu'en guanines et cytosines respectivement appariées mais, aussi, de la position relative de ces bases appariées dans une séquence donnée, dépend la température qui permet d'obtenir une hybridation spécifique donc une discrimination entre deux séquences qui ne diffèrent, entre elles, que d'une seule base.

La température qui permet une hybridation spécifique peut être calculée de façon approximative selon plusieurs formules qui tiennent compte de la longueur des séquences à apparier, ainsi que de leur composition en bases (voir en particulier Wallace et al., Nucleic Acids Res. 1979, 6(11):3543-3557 pour le calcul de la Tm selon la règle de Wallace, ainsi que Breslauer et al., Proc. Natl. Acad. Sci. USA, 1986, 83(11):3746-3750 pour le calcul de la Tm par la méthode du plus proche voisin). Cependant, aucune méthode de calcul ne permet de déterminer de façon précise la Tm d'un ADN double brin qui contient des dl puisque, dans toutes les formules actuellement proposées, l'énergie d'appariement entre dl, d'une part et dA, dG, ou dT, d'autre part, est considérée comme identique, ce qui est inexact (voir ci-dessous). Cette température peut également être déterminée expérimentalement par mesure de l'effet hyperchrome de l'ADN en fonction de la température. Pour cela la densité optique de l'ADN double brin est mesurée à 260 nm, en continu, en fonction de la température. Entièrement sous forme simple brin, l'ADN absorbe à 260 nm environ 1,4 fois plus que sous forme double brin. La température à laquelle l'ADN est, pour moitié, sous forme double brin et, pour moitié, sous forme simple brin est la température de fusion ou Tm. La Tm est mesurée au point d'inflexion de la courbe de dénaturation thermique de l'ADN qui représente la valeur de la densité optique mesurée à 260 nm en fonction de la température. Cette Tm est d'autant plus élevée que le nombre de guanines et cytosines appariées est important, dans une séquence donnée. Elle caractérise donc une séquence d'ADN double brin de longueur donnée selon sa composition en bases.

Déterminer la température optimale d'hybridation pour assurer une discrimination entre deux séquences qui ne diffèrent que d'un seul nucléotide et, à plus forte raison, de plusieurs nucléotides, est aisé, à condition que les séquences à apparier soient relativement courtes.

Cependant, une hybridation spécifique entre plusieurs sondes et autant de molécules d'acide nucléique (ADN ou ARN) qui ne diffèrent entre elles que d'un seul nucléotide ou qui présentent des séquences complètement différentes, avec des pourcentages différents en adénines et thymines (ou uraciles), ainsi qu'en guanines et cytosines respectivement appariées, requiert autant de conditions d'hybridation qu'il y a de séquences à hybrider. En effet, les conditions qui autorisent une hybridation spécifique dépendant de la composition en bases des séquences à hybrider, les températures nécessaires à l'obtention d'hybridations spécifiques seront d'autant plus élevées qu'augmentera le nombre de guanines et cytosines appariées en remplacement d'autant d'adénines et thymines (ou uraciles) appariées.

Par conséquent, il n'est pas possible d'obtenir, simultanément sur le même support solide, des hybridations spécifiques avec des séquences imposées de petite taille car les températures qui garantissent la spécificité d'hybridation devraient être différentes pour chaque séquence à hybrider, ce que confirme la détermination de leur Tm.

Cette impossibilité reste également vraie lorsque l'on désire effectuer ces hybridations spécifiques avec des séquences imposées de petite taille en milieu liquide dans un même réacteur, ou encore dans un ensemble de réacteurs auxquels on désire appliquer les mêmes conditions d'hybridations spécifiques, en particulier de température, comme par exemple un ensemble de cupules disposées sur une même plaque ou une même microplaque.

Ainsi, il reste souhaitable de pouvoir disposer aujourd'hui d'une méthode d'analyse permettant de réaliser des hybridations spécifiques entre acides nucléiques de séquences imposées de petite taille, séquences ne différant entre elles que d'un seul nucléotide ou séquences complètement différentes, autorisant des hybridations qui aboutissent à la formation de duplex présentant des pourcentages différents en adénines et thymines (ou uraciles), ainsi qu'en guanines et cytosines respectivement appariées, ceci simultanément sur un même support solide, ou encore en phase liquide dans un même réacteur ou dans un ensemble de réacteurs auxquels on désire appliquer les mêmes conditions d'hybridation spécifique, en particulier de température.

Ceci est justement l'objet de la présente description.

Le problème, dont la solution est apportée par la présente description, est de pouvoir définir un ensemble de sondes nucléiques dont on pourra disposer et dont la composition en nucléotides permet d'obtenir des conditions similaires d'hybridation pour cet ensemble de sondes, telles que ces sondes soient susceptibles de s'hybrider de façon spécifique à des acides nucléiques cibles de type ADN et/ou ARN de séquence imposée, quel que soit leur pourcentage relatif en adénines et thymines, ainsi qu'en guanines et cytosines.

Pour homogénéiser la température d'hybridation de toutes les sondes avec des acides nucléiques cibles (ADN ou ARN) de séquence imposée, tout en gardant une spécificité absolue d'hybridation, quel que soit le pourcentage relatif en adénines et thymines, ainsi qu'en guanines et cytosines respectivement appariées, il est proposé ici d'utiliser des sondes courtes qui possèdent des désoxyinosines (dl) au lieu de (dG) ceci non seulement pour assurer un appariement spécifique avec des dC ou des C, mais également pour assurer cet appariement spécifique avec des dC ou des C par l'intermédiaire de deux liaisons hydrogènes, à la place des trois liaisons hydrogènes formées entre dG et dC ou dG et C.

Le nombre et la position des dl qui remplacent les dG dans les séquences des sondes imposées sont déterminés de façon à ce que les séquences des sondes imposées ainsi modifiées présentent entre elles un contenu similaire, de préférence égal, en guanines et cytosines, l'hybridation de ces sondes modifiées aboutissant ainsi à la formation de duplex ADN/ADN ou d'hétéroduplex ADN/ARN dont les Tm sont suffisamment voisines pour autoriser des conditions d'hybridation spécifique identiques pour chacune de ces sondes.

Le nombre et la position des dl qui remplacent les dG seront en particulier déterminés par le nombre et la position à l'intérieur de la séquence d'une sonde donnée, des appariements entre dG et dC ou dG et C, qui remplacent des appariements entre dA et dT ou dA et U d'une autre séquence sonde donnée.

Par la solution proposée ici, la dl n'est pas utilisée en tant que nucléotide susceptible de s'apparier aussi bien avec dC qu'avec dA, dG et dT ou, aussi bien avec C qu'avec A, G et U. En effet, si la dl est bien susceptible de s'apparier avec ces désoxyribonucléosides et ribonucléosides, toujours par l'intermédiaire de deux liaisons hydrogènes, l'énergie d'appariement est très différente selon les cas. Par ordre décroissant d'énergie d'appariement, on aura dl:dC > dl:dA > dl:dG = dl:dT et dl:C > dl:A > dl:G = dl:U. De plus, en fonction de l'environnement de dl, l'énergie d'appariement n'est pas strictement identique, selon que cette dl est positionnée avant ou après dA, dG, dC, dT ou une autre dl. Néanmoins, dans tous les cas, dl s'appariera toujours mieux avec dC ou C qu'avec dT ou U, ces deux dernières possibilités d'hybridation étant toujours les moins favorables. Sur les propriétés d'appariement de dl avec les autres désoxyribonucléotides, on pourra mais sans s'y limiter, se référer aux articles de Case-Green et al. (Nucleic Acids Res., 1994, 22(2):131-136) et de Martin et al. (Nucleic Acids Res., 1995, 13(24):8927-8938) et aux documents qui y sont référencés.

Ainsi, sous un premier aspect, la présente description a pour objet une méthode d'analyse d'un échantillon contenant un polynucléotide cible (à l'intérieur d'une molécule d'ADN ou d'ARN) et/ou l'un de ses variants, ledit variant comprenant une ou plusieurs substitutions de base par rapport audit polynucléotide cible, ladite méthode comprenant les étapes suivantes :
a) la mise à disposition de l'échantillon contenant ledit polynucléotide cible, ou l'un de ses variants, et d'au moins deux oligodésoxyribonucléotides différents, ci-après désignés par le terme général de "sondes", ces dernières étant disposées soit de manière distincte sur un support solide, soit contenues dans un même réacteur, soit encore réparties de manière distincte sur un dispositif comprenant un ensemble de réacteurs, chacune desdites sondes présentant une séquence susceptible de former un duplex spécifique avec le polynucléotide cible ou l'un de ses variants attendus ;
b) l'incubation dudit polynucléotide cible, ou l'un de ses variants, avec lesdites sondes dans des conditions d'hybridation spécifique, permettant d'obtenir la formation d'un duplex entre ledit polynucléotide cible ou l'un de ses variants attendus, et une desdites sondes, même si ledit polynucléotide cible et l'un de ses variants attendus ne diffèrent entre eux que d'un seul nucléotide ; et
c) la détection des duplex formés sur ledit support solide, ou encore formés en solution dans ledit même réacteur ou formés en solution dans chacun des réacteurs dudit ensemble de réacteurs,
caractérisée en ce qu'au moins un des nucléotides dG dans au moins une desdites sondes, a été substitué par un nucléotide dl, de telle façon que les conditions d'hybridation spécifique permettant d'obtenir la formation d'un duplex entre ledit polynucléotide cible, ou l'un de ses variants attendus, et une desdites sondes à l'étape b) sont identiques pour chacune desdites sondes.

De préférence, une méthode d'analyse est caractérisée en ce que à l'étape a) lesdites au moins deux sondes différentes sont disposées de manière distincte sur un support solide, soit contenues en solution dans un même réacteur, soit encore réparties en solution de manière distincte sur un dispositif comprenant un ensemble de mêmes réacteurs, et en ce qu'à l'étape c), respectivement, les duplex formés sont détectés sur le support solide, les duplex formés en solution sont détectés dans ledit même réacteur (ou partir d'une aliquote contenue dans ce même réacteur) ou dans chacun des réacteurs dudit ensemble de même réacteurs.

Par ensemble de réacteurs, on entend désigner dans la présente description, un ensemble de réacteurs, de préférence de mêmes réacteurs, de préférence encore regroupés dans un ou, suivant le nombre de réacteurs, dans plusieurs mêmes dispositifs, et où les mêmes conditions d'hybridation seront appliqués pour chacun des réacteurs de cet ensemble.

Dans un mode de réalisation préféré, lorsque le nombre de réacteurs est supérieur à un, le nombre de réacteurs est égal au nombre desdites sondes différentes.

De préférence encore, cet ensemble de réacteurs pourra se présenter, suivant la taille et/ou le nombre des réacteurs, sous la forme d'une ou plusieurs mêmes plaques ou microplaques sur lesquelles seront disposées des conteneurs, cupules ou puits dans lesquels l'incubation dudit polynucléotide cible, ou l'un de ses variants, avec lesdites sondes sera effectuée en milieu liquide.

Sous ce premier aspect, une méthode d'analyse d'un échantillon contenant un polynucléotide cible et/ou l'un de ses variants est décrite, ledit variant comprenant une ou plusieurs substitutions de base par rapport audit polynucléotide cible, ladite méthode comprenant les étapes suivantes :
a) la mise à disposition de l'échantillon contenant ledit polynucléotide cible, ou l'un de ses variants, et d'au moins deux oligodésoxyribonucléotides différents, ci-après désignés par le terme général de "sondes", ces dernières étant disposées de manière distincte sur un support solide, chacune desdites sondes présentant une séquence susceptible de former un duplex spécifique avec le polynucléotide cible ou l'un de ses variants attendus ;
b) l'incubation dudit polynucléotide cible, ou l'un de ses variants, avec lesdites sondes dans des conditions d'hybridation spécifique, permettant d'obtenir la formation d'un duplex entre ledit polynucléotide cible, ou l'un de ses variants attendus, et une desdites sondes, même si ledit polynucléotide cible et l'un de ses variants attendus ne diffèrent entre eux que d'un seul nucléotide ; et
c) la détection des duplex formés sur le support solide,
caractérisée en ce qu'au moins un des nucléotides dG dans au moins une desdites sondes, a été substitué par un nucléotide dl, de telle façon que les conditions d'hybridation spécifique permettant d'obtenir la formation d'un duplex entre ledit polynucléotide cible, ou l'un de ses variants attendus, et une desdites sondes à l'étape b) sont identiques pour chacune desdites sondes.

Par acide nucléique, sonde nucléique, séquence nucléique ou d'acide nucléique, polynucléotide, oligonucléotide, séquence de polynucléotide, séquence nucléotidique, séquence polynucléotidique et, en absence d'autre précision, on entend désigner ici un enchaînement précis de nucléotides, modifiés ou non, permettant de définir un fragment ou une région d'un acide nucléique, comportant ou non des nucléotides autres que dA, dT, dG, dC ou A, U, G et C, et pouvant correspondre aussi bien à un ADN double brin ou un ADN simple brin qu'à des produits de transcription desdits ADN tels que des ARN.

Par sonde, on entendra désigner ici un oligodésoxyribonucléotide utilisé en tant que sonde.

Par sondes de petite taille, on entendra désigner ici des sondes dont la taille est suffisamment courte pour permettre l'hybridation spécifique et la discrimination entre deux polynucléotides cibles différents, même si cette différence ne porte que sur une base. De préférence, ces sondes présenteront une longueur inférieure à 40 nucléotides et, de façon encore plus préférée, inférieure à 35, 30, 25, 24, 23, 22, 21 nucléotides. De préférence encore, lesdites sondes comprendront, au moins, 10 nucléotides et, de façon encore plus préférée, 11, 12, 13, 14, 15, 16, 17, 18, 19 ou 20 nucléotides.

Par oligonucléotide cible on entendra désigner ici un oligonucléotide que l'on cherche à détecter et/ou identifier et/ou quantifier dans un échantillon, ceci par opposition au terme de sonde, oligonucléotide cible généralement dérivé d'une partie intégrante d'un ADN ou d'un ARN extrait d'un prélèvement biologique, de tissu, de cellules ou de microorganismes tels que des bactéries, levures, champignons, algues ou encore de virus ou phages.

Par oligonucléotide cible dérivé d'un ADN ou d'un ARN, on entendra désigner ici un oligonucléotide (ADN simple brin ou ARN simple brin) dont la séquence est identique ou complémentaire à 100 % de la séquence dudit ADN, ou dudit ARN, ou de la séquence d'un de leurs fragments.

Par variant ou variant attendu d'un oligonucléotide cible, on entendra désigner ici un oligonucléotide dont la séquence comporte une ou plusieurs substitutions de base par rapport audit oligonucléotide cible, variant dont on cherche à détecter et/ou identifier et/ou quantifier la présence dans un échantillon.

Par duplex, on entend désigner dans la présente description un acide nucléique double brin résultant de l'hybridation spécifique entre deux brins d'ADN complémentaires, strictement ou non (homoduplex ADN/ADN) ou entre un brin d'ADN et un brin d'ARN qui lui est complémentaire, strictement ou non (hétéroduplex ADN/ARN). Dans la présente description, seuls les nucléotides dC ou C seront considérés comme étant complémentaires de dl.

Par édition de l'ARN ("RNA editing") ou, plus simplement, par édition, on entend désigner dans la présente description, la désamination des adénosines (A) en inosines (I), à l'intérieur d'une séquence d'ARN, par des adénosines désaminases dont l'activité dépend de l'ARN double brin ; ces adénosines désaminases sont des ADAR ("adenosine deaminase RNA-dependent") selon que l'ARN cible est un ARNm, ou bien des ADAT ("adenosine deaminase tRNA-dependent") selon que les ARN cibles sont des ARNt (pour revue, voir Maas et al. BioEssays, 2000, 22(9), 790-802, ainsi que Reenan, TRENDS in Genetics, 2001, 17(2), 53-56, ainsi que Gerber et al. TRENDS in Biochemical Sciences, 2001, 26(6), 376-384, ainsi que Baas, Ann. Rev. Biochem. 2002, 71,817-846, avec les articles qui y sont référencés).

Par ARN édité, on entend désigner dans la présente description, toute séquence d'ARN dont, au moins, une adénosine a été désaminée en inosine par une adénosine désaminase.

Dans un mode de réalisation préféré, le nombre et la localisation des substitutions de nucléotide dG par un nucléotide dl sur lesdites sondes sont déterminés de manière à ce que les températures de fusion (Tm) des duplex pouvant être formés entre une desdites sondes et ledit polynucléotide cible, ou l'un de ses variants attendus, sont identiques ou suffisamment proches pour permettre l'obtention desdits duplex par hybridation spécifique dans les mêmes conditions d'hybridation pour chacune desdites sondes. De préférence, les écarts maximum des Tm entre lesdits duplex seront, au maximum, de 10°C et, de façon encore plus préférée, de 9, 8, 7, 6, 5°C ou moins.

Les méthodes permettant de mesurer les Tm d'ADN double brin sont anciennes et bien connues de l'homme de l'art. Elles ne seront pas développées ici mais on peut citer pour mémoire Lehman et al. (J. Chem. Phys. 1968, 4(7):3170-3179), Crothers (Biopolymers, 1968, 6(10):1391-1404) et, pour revue, Lazurlin et al. (Biopolymers, 1970,9(11):1253-1306).

Dans un mode de réalisation également préféré, le nombre et la localisation des substitutions de nucléotide dG par un nucléotide dl sur lesdites sondes sont déterminés de manière à ce que le nombre de dG restants susceptibles de s'apparier à dC ou à C dans lesdits duplex est, de préférence, identique ou peu différent pour chacune desdites sondes. De préférence encore, la différence entre le nombre de dG restants susceptibles de s'apparier à dC ou à C dans lesdits duplex, est inférieure à 10% de la longueur total dudit duplex.

Dans un mode de réalisation également préféré, ledit polynucléotide cible ou l'un de ses variants attendus est de type ADN simple brin ou ARN simple brin.

Dans un mode de réalisation également préféré, lesdites sondes comprennent un petit nombre de bases, de préférence 30 bases ou moins de 30 bases, de manière préférée 25 bases ou moins de 25 bases, de manière encore plus préférée 20 bases ou moins de 20 bases.

Dans un mode de réalisation également préféré, lesdites sondes et lesdits polynucléotides cibles, ou l'un de leurs variants attendus, mettent en jeu un nombre identique de bases ou un nombre de bases différant d'une unité ou, au plus, de 10 %.

Dans un mode de réalisation également préféré, ledit polynucléotide cible, ou l'un de ses variants attendus, est préalablement marqué par un marqueur capable d'engendrer, directement ou indirectement un signal détectable, de préférence détectable par fluorescence ou par mesure de radioactivité lorsque lesdites sondes sont disposées sur un support solide.

Par exemple, lesdits marqueurs lorsqu'ils sont fluorescents peuvent être choisis parmi des fluorochromes, notamment dérivés de la cyanine, tels que les fluorochromes Cy5 ou Cy3.

Dans un mode de réalisation également préféré, l'appariement spécifique de la sonde avec ledit polynucléotide cible est mis en évidence grâce à l'utilisation d'un composé susceptible d'être détecté par transfert d'énergie moléculaire, notamment par transfert d'énergie de fluorescence (dénommé « FRET » pour "fluorescence resonance energy transfer").

Par la technique « FRET », l'appariement spécifique de la sonde avec ledit polynucléotide cible ou l'un de ses variants pourra, par exemple, être révélé par un couple de fluorophores, le premier fluorophore étant soit accepteur soit donneur de photons et situé en 5' ou en 3' de la sonde, le second fluorophore étant alors respectivement soit donneur soit accepteur de photons, et positionné respectivement en 3' ou en 5' d'une seconde sonde ; cette seconde sonde est définie telle qu'elle puisse s'hybrider de façon strictement identique à une séquence identique et adjacente auxdits polynucléotides cibles, et telle que le second fluorophore, donneur ou accepteur de photon, soit positionné à distance suffisamment proche du premier fluorophore, pour assurer le transfert d'énergie du fluorophore donneur au florophore accepteur.

Dans les méthodes d'analyse ou procédés décrits, les marqueurs et les techniques permettant de détecter et/ou de quantifier la présence de duplex formés sur un support solide revêtu de sondes, ou formés en phase homogène liquide sont bien connus de l'homme de l'art et ne seront pas développés ici.

Pour les systèmes de marqueurs permettant la détection d'une hybridation entre deux polynucléotides en phase liquide homogène par transfert d'énergie moléculaire, notamment par « FRET », on pourra par exemple, mais sans s'y limiter, se référer à l'article de Livak et al. (PCR Methods Appl. 1995, 4:357-362) et au document brevet US 6,117,637 et aux documents qui y sont référencés.

Dans un mode de réalisation encore préféré, ledit support solide est un support solide pouvant être, le cas échéant, activé ou fonctionnalisé pour assurer une fixation covalente desdites sondes, notamment choisis parmi les supports solides en verre, en plastique, en Nylon^{®}, en silicone, en silicium, ou encore en polyoses ou poly(hétéro-oses), tel que la cellulose, de préférence en verre, ce dernier pouvant être silanisé.

Dans un mode de réalisation également préféré, lesdites sondes pourront être fonctionnalisées à l'aide d'une fonction capable de réagir avec ledit support solide pour former une liaison covalente avec ledit support, ce dernier pouvant être activé, fonctionnalisé ou encore muni d'un agent espaceur.

Dans un mode de réalisation également préféré, ledit échantillon à analyser contient ledit polynucléotide cible et l'un au moins de ses variants attendus.

Dans un mode de réalisation également préféré, le nombre desdites sondes différentes est au moins égal à 3, 4, 5, 6 7, 8, 9, 10, 15, 20 et 25 ou encore au moins égal au nombre de variants attendus dudit polynucléotide cible dont l'un au moins est susceptible d'être présent dans ledit échantillon à analyser, ledit nombre de variants étant au moins égal à 2.

Dans un mode de réalisation également préféré, le nombre desdites sondes dont au moins un des nucléotides dG a été substitué par un nucléotide dl est au moins égal à 2, de préférence à 3, 4, 5, 6, 7, 8, 9, 10, 15 ou encore au nombre de variants attendus dudit polynucléotide cible comportant un nucléotide dC ou C à la place d'un autre nucléotide présent dans ledit polynucléotide cible.

Sous un autre aspect, un jeu d'au moins deux sondes oligodésoxyribonucléotidiques différentes est décrit, caractérisé en ce qu'au moins un des nucléotides dG sur au moins une desdites sondes a été substitué par un nucléotide dl, de telle façon que les conditions d'hybridation sont identiques pour chacune desdites sondes, ces dernières contenant une séquence susceptible de former un duplex spécifique avec un polynucléotide cible, ou l'un de ses variants.

De préférence, le jeu d'au moins deux sondes différentes est caractérisé en ce que le nombre et la localisation des substitutions de nucléotide dG par un nucléotide dl sur lesdites sondes sont déterminés de manière à ce que les Tm des duplex pouvant être formés avec chacune desdites sondes sont identiques ou suffisamment proches pour permettre l'obtention desdits duplex par hybridation spécifique dans les mêmes conditions d'hybridation pour chacune desdites sondes.

De préférence encore, le jeu d'au moins deux sondes différentes est caractérisé en ce que le nombre et la localisation des substitutions de nucléotide dG par un nucléotide dl sur lesdites sondes sont déterminés de manière à ce que le nombre de dG restants susceptibles de s'apparier à dC ou à C dans lesdits duplex est identique ou peu différent pour chacune desdites sondes.

De préférence encore, le jeu d'au moins deux sondes différentes est caractérisé en ce que lesdites sondes sont de type ADN monocaténaire.

De préférence encore, le jeu d'au moins deux sondes différentes est caractérisé en ce que lesdites sondes comprennent un petit nombre de bases, de préférence 30 bases ou moins de 30 bases, de manière préférée 25 bases ou moins de 25 bases, de manière encore plus préférée 20 bases ou moins de 20 bases.

De préférence encore, le jeu d'au moins deux sondes différentes est caractérisé en ce que lesdites sondes mettent en jeu un nombre identique de bases ou différant d'une unité ou, au plus, de 10 %.

Dans un mode de réalisation également préféré, le nombre desdites sondes différentes est au moins égal à 3, 5, 7, 10, 15, 20 et 25 ou encore au moins égal au nombre de variants attendus dudit polynucléotide cible dont l'un au moins est susceptible d'être présent dans ledit échantillon à analyser, ledit nombre de variants étant au moins égal à 2.

De préférence encore, le jeu d'au moins deux sondes différentes est caractérisé en ce que le nombre desdites sondes dont au moins un des nucléotides dG a été substitué par un nucléotide dl est au moins égal à 2, de préférence à 3, 4, 5, 6, 7, 8, 9, 10, 15 ou encore au nombre de variants attendus dudit polynucléotide cible comportant un nucléotide dC ou C à la place d'un autre nucléotide présent dans ledit polynucléotide cible.

Sous un aspect particulièrement préféré, un jeu d'au moins deux sondes différentes est décrit, caractérisé en ce que ledit jeu est constitué ou comprend un ensemble de sondes permettant de détecter et/ou de quantifier dans un échantillon, la présence d'un oligonucléotide cible et un ensemble de ses variants attendus.

De préférence, ledit oligonucléotide cible est dérivé d'un fragment de gène susceptible de comporter - ou dont le produit de transcription est susceptible de comporter - au moins une substitution de base dans la séquence dont dérive ledit oligonucléotide cible.

De préférence encore, ledit oligonucléotide cible est dérivé d'un fragment d'ARNm susceptible de comporter au moins un site d'édition dans la séquence de ce fragment.

De manière encore plus préférée, ledit oligonucléotide cible est dérivé d'un fragment d'ARNm d'un récepteur membranaire de cellule eucaryote, notamment de mammifère, incluant le récepteur de la sérotonine 5-HT_{2C} (5-HT_{2C}-R) ou la sous-unité B du récepteur du glutamate (GluR-B), le 5-HT_{2C}-R étant le plus préféré.

Dans un mode de réalisation également préféré, un jeu d'au moins deux sondes différentes est décrit, caractérisé en ce que ledit jeu est constitué d'un ou comprend un ensemble de sondes distinctes permettant de détecter et/ou de quantifier dans un échantillon, la présence potentielle de tout oligonucléotide cible dérivé d'un fragment d'ARNm, édité et non édité.

Dans un mode de réalisation particulièrement préféré, un jeu d'au moins deux sondes différentes est décrit, caractérisé en ce que ledit jeu est constitué d'un ou comprend au moins un ensemble de sondes distinctes permettant de détecter et/ou de quantifier dans un échantillon, la présence potentielle du polynucléotide cible dérivé du fragment d'ARNm non édité et de tout polynucléotide cible dérivé d'un fragment d'ARNm dont l'édition peut entraîner une modification de la séquence d'acides aminés de la protéine qui est le produit de traduction de l'ARNm non édité.

Dans un mode de réalisation encore plus préféré, un jeu d'au moins deux sondes différentes est décrit, caractérisé en ce que ledit jeu est constitué d'un ou comprend un ensemble de trente-deux sondes distinctes permettant de détecter et/ou de quantifier dans un échantillon tout oligonucléotide cible dérivé d'un fragment comprenant la séquence SEQ ID No. 33 (5'-AUA CGU AAU CCU A-3') de l'ARNm, édité ou non édité, du 5-HT_{2C}-R.

Dans ce cas particulier, on entend désigner par "tout oligonucléotide cible dérivé d'un fragment de l'ARNm du 5-HT_{2C}-R, ledit fragment comprenant la séquence SEQ ID No. 33 (5'-AUA CGU AAU CCU A-3') de l'ARNm édité ou non édité" :
- n'importe lequel des oligonucléotides de l'ensemble des oligonucléotides constitué par les trente-deux ARN antisens, c'est-à-dire complémentaires à 100 % du fragment de l'ARNm du 5-HT_{2C}-R, ledit fragment comprenant la séquence SEQ ID No. 33 et dans laquelle SEQ ID No. 33, chaque nucléotide A correspondant à un site d'édition (nucléotides en position 1,3, 7, 8, ou 13 de la SEQ ID No. 33) peut être édité ou non ; ou
- n'importe lequel des oligonucléotides de l'ensemble des oligonucléotides constitué par les trente-deux ADN, c'est-à-dire complémentaires à 100 % du fragment de l'ARNm du 5-HT_{2C}-R, ledit fragment comprenant la séquence SEQ ID No. 33 et dans laquelle SEQ ID No. 33, chaque nucléotides A correspondant à un site d'édition (nucléotides en position 1,3, 7, 8, ou 13 de SEQ ID No. 33) peut être édité ou non.

Dans un mode de réalisation encore plus préféré, un jeu d'au moins deux sondes différentes est décrit, caractérisé en ce que ledit jeu est constitué ou comprend les ensembles de sondes suivants :
- l'ensemble des trente-deux sondes de séquence comprenant les séquences des fragments du nucléotide 3 au nucléotide 15 des SEQ ID Nos. 1 à 32, les parties de ces sondes situées en dehors dudit fragment du nucléotide 3 au nucléotide 15 étant identiques aux parties correspondantes (en ADN) de l'ARNm du 5-HT_{2C}-R ; ou
- l'ensemble des trente-deux sondes de séquence SEQ ID Nos. 1 à 32.

Sous un autre aspect, une biopuce comprenant un jeu d'au moins deux sondes différentes , déposées sur un même support solide est décrite, ledit support solide pouvant être, le cas échéant, activé ou fonctionnalisé pour assurer une fixation covalente desdites sondes, et notamment choisi parmi les supports solides tels que décrits ci-avant.

Sous un autre aspect, un réacteur comprenant en solution un jeu d'au moins deux sondes différentes est décrit.

Sous un autre aspect, un dispositif, notamment une plaque ou une microplaque, constitué d'au moins deux conteneurs ou cupules est décrit, ledit dispositif comprenant un jeu d'au moins deux sondes différentes selon la présente description, chacun des conteneurs ou cupules contenant une desdites sondes.

De préférence, une biopuce dans laquelle lesdites sondes sont fixées sur ledit support solide est décrite, notamment par liaison covalente.

Sous un autre aspect, une trousse de réactifs pour la détection, l'analyse qualitative ou quantitative d'acides nucléiques cibles dans un échantillon est décrite, caractérisé en ce qu'elle comprend un jeu d'au moins deux sondes différentes selon la présente invention et/ou une biopuce selon la description

Sous encore un autre aspect, un procédé pour la détection et/ou la quantification d'oligonucléotides cibles dans un échantillon est décrit, caractérisé en ce qu'il comprend les étapes suivantes :
a) le dépôt de l'échantillon contenant lesdits oligonucléotides cibles dont on cherche à détecter la présence sur une biopuce selon la description, ou encore dans chacun des conteneurs ou cupules du dispositif selon la description, dans les conditions permettant l'hybridation spécifique desdits oligonucléotides cibles avec lesdites sondes ;
b) le cas échéant, le rinçage de la biopuce obtenue à l'étape a) dans les conditions permettant l'élimination des acides nucléiques de l'échantillon non capturés par hybridation ; et
c) la détection et/ou la quantification des oligonucléotides cibles capturés par chacune desdites sondes par hybridation spécifique.

Un procédé pour la détection et/ou la quantification d'oligonucléotides cibles dans un échantillon est également décrit, ledit oligonucléotide cible étant dérivé d'un fragment d'ARNm susceptible de comporter au moins un site d'édition dans la séquence du fragment dont dérive ledit oligonucléotide cible, caractérisé en ce qu'il comprend les étapes suivantes :
a) le dépôt de l'échantillon contenant lesdits oligonucléotides cibles dont on cherche à détecter la présence sur une biopuce revêtue d'un jeu de sondes selon l'invention, ou encore dans chacun des conteneurs ou cupules du dispositif selon l'invention, dans les conditions permettant l'hybridation spécifique desdits oligonucléotides cibles avec lesdites sondes ;
b) le cas échéant, le rinçage de la biopuce obtenue à l'étape a) dans les conditions permettant l'élimination des acides nucléiques de l'échantillon non capturés par hybridation ; et
c) la détection et/ou la quantification des oligonucléotides cibles capturés par chacune desdites sondes par hybridation spécifique.

Un procédé pour la détection et/ou la quantification de tout oligonucléotide cible dérivé d'un fragment d'ARNm susceptible de comporter au moins un site d'édition dans la séquence du fragment dont dérive ledit oligonucléotide cible est également décrit, caractérisé en ce qu'il comprend les étapes suivantes :
a) le dépôt de l'échantillon contenant lesdits oligonucléotides cibles dont on cherche à détecter la présence,
   - soit sur une biopuce revêtue d'un jeu de sondes selon la description, ou encore
   - soit dans chacun des conteneurs ou cupules du dispositif selon la description, notamment dans chacune des cupules d'une microplaque, constitué d'au moins deux cupules, ladite microplaque comprenant un jeu de sondes selon la présente description, chacune des cupules contenant une seule desdites sondes,
   dans les conditions permettant l'hybridation spécifique desdits oligonucléotides cibles avec lesdites sondes, ledit jeu de sondes étant constitué d'un ou comprenant un ensemble de sondes distinctes capable de détecter et/ou de quantifier dans un échantillon la présence potentielle de tout oligonucléotide cible dérivé d'un fragment d'ARNm, édité et non édité ;
b) le cas échéant, le rinçage de la biopuce obtenue à l'étape a) dans les conditions permettant l'élimination des acides nucléiques de l'échantillon non capturés par hybridation ; et
c) la détection et/ou la quantification des oligonucléotides cibles capturés par chacune desdites sondes par hybridation spécifique.

La présente description comprend également un procédé pour la détection et/ou la quantification de tout oligonucléotide cible présent dans un échantillon et dérivé d'un fragment comprenant la séquence SEQ ID No. 33 (5'-AUA CGU AAU CCU A-3') de l'ARNm édité ou non édité, du 5-HT_{2C}-R, caractérisé en ce qu'il comprend les étapes suivantes :
a) le dépôt de l'échantillon contenant lesdits oligonucléotides cibles dont on cherche à détecter la présence,
   - soit sur une biopuce revêtue d'un jeu constitué d'un ou comprenant l'ensemble de trente-deux sondes distinctes selon la description, ou encore
   - soit dans chacun des conteneurs ou cupules du dispositif selon la description, ce dispositif comprenant un jeu constitué d'un ou comprenant l'ensemble de trente-deux sondes distinctes selon la description, notamment dans chacune des cupules d'une microplaque comprenant 32 cupules, chacune de ces cupules contenant une seule desdites sondes de l'ensemble de trente-deux sondes,
   l'ensemble des trente-deux sondes permettant de détecter et/ou de quantifier dans un échantillon tout oligonucléotide cible dérivé d'un fragment de l'ARNm du 5-HT_{2C}-R, ledit fragment comprenant la séquence SEQ ID No. 33 (5'-AUA CGU AAU CCU A-3') édité ou non édité, dans les conditions permettant l'hybridation spécifique desdits oligonucléotides cibles avec lesdites sondes ;
b) le cas échéant, le rinçage de la biopuce obtenue à l'étape a) dans les conditions permettant l'élimination des acides nucléiques de l'échantillon non capturés par hybridation ; et
c) la détection et/ou la quantification des oligonucléotides cibles capturés par chacune desdites sondes par hybridation spécifique.

Sous encore un autre aspect, un procédé de détermination, dans un échantillon, du pourcentage de chacune des formes éditées et non éditées de l'ARNm susceptible de comporter au moins un suite d'édition, par rapport à la quantité totale des formes d'ARNm éditées ou non éditées présentes dans ledit même échantillon (dénommé « taux d'édition») est également décrit, caractérisé en ce qu'il comprend un procédé selon la description pour la détection et/ou la quantification de tout oligonucléotide cible présent dans un échantillon et dérivé d'un fragment de l'ARNm susceptible d'être édité ou non, et dans lequel procédé à la fin de l'étape c) on détermine pour chacune desdites sondes le rapport exprimé en pourcentage entre la quantité d'oligonucléotides capturés par une sonde et la quantité totale d'oligonucléotides capturés par l'ensemble des sondes.

Dans un mode particulièrement préféré des procédés ci-avant selon la description, les oligonucléotides cibles sont des ARNm antisens ou des ADN complémentaires des fragments dudit ARNm édité ou non édité, et lesdites sondes sont des ADN correspondant aux séquences du fragment dudit ARNm édité ou non édité.

Dans un mode également préféré des procédés ci-avant, lesdits fragments dont dérivent les oligonucléotides cibles sont des fragments d'acides nucléiques extraits à partir d'un prélèvement biologique d'un organisme eucaryote, notamment le mammifère, dont l'être humain.

Dans un mode de réalisation également préféré, les oligonucléotides cibles mis en oeuvre dans les procédés selon la description sont préalablement marqués par un marqueur capable d'engendrer directement ou indirectement un signal détectable, de préférence fluorescent ou radioactif.

Sous encore un autre aspect, l'utilisation d'une biopuce ou d'un dispositif selon la description comme matrice d'affinité, pour la purification des acides nucléiques ou pour le séquençage des acides nucléiques, est également décrite.

L'utilisation d'une biopuce ou d'un dispositif selon la description pour la détection et/ou l'étude de polymorphismes génétiques, notamment de SNP ("Single Nucleotide Polymorphism") ou pour l'analyse qualitative ou quantitative de l'expression de gènes, est également décrite.

Par exemple, mais sans s'y limiter, on pourra déposer sur les biopuces ou dans les conteneurs, notamment des cupules des dispositifs selon la description, des sondes d'ADN correspondant à un gène d'intérêt connu. Chaque dépôt pourra contenir une sonde correspondant à un gène et, d'un dépôt à l'autre, les sondes correspondront audit gène présentant un polymorphisme.

Les ADN ou ARN génomiques, ou ARNm, ou leurs fragments, du tissu, de la cellule ou des microorganismes que l'on souhaite étudier pourront être extraits puis marqués par exemple avec des fluorochromes ou avec des composés radioactifs (les ADN pourront être amplifiés par PCR et les ARN ou ARNm pourront notamment être transformés en ADN complémentaires (ADNc) par transcription inverse et, le cas échéant, amplifiés par les techniques de RT-PCR, ou encore transformés en ARN antisens).

Ces ADNc ou ARN antisens seront ensuite déposés sur la biopuce revêtue des sondes selon la description et, le cas échéant, pourront se lier par hybridation spécifique avec ces sondes préalablement déposées qui leur correspondent. Sera ensuite détectée sur chaque dépôt ou dans chaque conteneur, la quantité de signal, notamment de fluorescence ou de radioactivité, correspondant ainsi à la quantité d'acides nucléiques cibles hybridés, qui sera notamment proportionnelle à la quantité initiale d'ARNm extraits, si les ADN cibles déposés sont des ADNc complémentaires d'ARNm rétro-transcrits. On pourra ainsi mesurer l'activité de transcription de la cellule pour certains gènes.

Les applications de ces biopuces à ADN ou dispositifs sont par conséquent nombreuses, telles que les études de la transcription, le diagnostic (recherche de mutation), la recherche de cibles thérapeutiques, le génotypage.

L'utilisation d'une biopuce ou d'un dispositif selon la description pour la détermination du taux d'édition d'un ARNm est également décrite.

Les méthodes d'analyse par des procédés utilisant des biopuces ou des dispositifs selon la description dans des trousses de réactif selon la description permettent de mettre en évidence une ou plusieurs substitutions de base dans une courte séquence d'ADN. Elles permettent également de révéler les polymorphismes d'un seul nucléotide (SNP). Elles permettent aussi de mesurer le degré d'édition des ARNm, que cette édition soit restreinte à un seul site ou qu'elle affecte plusieurs sites dans la même molécule d'ARNm. Autrement dit, elles permettent de révéler tout mésappariement, de un à plusieurs nucléotides, dans une courte séquence donnée quelle que soit sa composition en bases. Par cette caractéristique, elles permettent donc de poser le diagnostic de mutation ponctuelle, comme elles permettent de détecter la présence d'agents pathogènes - dont elles permettent le génotypage - qui peuvent être des virus, des procaryotes ou des eucaryotes, qui infectent l'homme, les animaux et les plantes. Elles permettent aussi l'analyse du transcriptome avec des sondes courtes, selon une stratégie différente de celles utilisées jusqu'ici, puisqu'elle n'impose pas, entre les différentes séquences, un pourcentage constant en guanines et cytosines appariées. Elles permettent enfin d'assurer le génotypage d'individus appartenant soit à la même espèce, soit à des espèces différentes, dans le cadre de la génomique comparative.

Sous un autre aspect, l'invention a pour objet une méthode SSCP permettant dans des conditions données d'analyse d'obtenir le profil d'édition de l'ARNm du 5-HT_{2C}-R , à partir d'un échantillon de tissu spécifique ou à partir d'un échantillon d'une population de cellules eucaryotes, caractérisée en ce qu'elle comprend les étapes suivantes :
a) l'extraction des ARN totaux dudit échantillon, suivie le cas échéant d'une purification des ARNm ;
b) la transcription inverse des ARN extraits à l'étape a) et la synthèse de l'ADN double brins ;
c) l'amplification par PCR des ADN obtenus à l'étape b) à l'aide d'un couple d'amorces spécifiques dudit ARNm pouvant être édité, ce couple d'amorces étant choisi de manière à pouvoir amplifier toutes les formes d'édition potentiellement présentes dans l'extrait d'ARN, ces amorces étant marquées par des fluorophores ;
d) le cas échéant, la purification des produits de PCR obtenus à l'étape c) ;
e) le cas échéant, la quantification des produits de PCR obtenus à l'étape d) ;
f) la dissociation des ADN double brins en ADN simple brin, notamment par chauffage suivi d'un refroidissement brutal ;
g) la séparation des ADN simple brin par électrophorèse capillaire ; et
h) l'obtention du profil de migration électrophorétique des différents ADN simple brin, dénommée ici « profil d'édition » par lecture de la fluorescence et, le cas échéant, acquisition des données du profil par le système d'exploitation associé au lecteur en fluorescence.

Par « méthode SSCP », on entendra désigner dans la présente description une méthode fondée sur la mise en évidence de polymorphisme de conformation de l'ADN simple brin (« SSCP » pour « Single Strand Conformational Polymorphism »).

De préférence dans la méthode SSCP selon l'invention, le couple d'amorces utilisées à l'étape c) est choisi de manière à ce que les produits PCR obtenus sont de longueur d'au moins 100 bases, de manière plus préférée d'au moins, 125, 150, 175, 200, 225 ou 250 bases, ceci afin de permettre un repliement caractéristique de la forme d'édition de chacun ou des deux brins séparés après l'étape g).

De préférence dans la méthode SSCP décrite, ledit ARNm pouvant être édité est celui d'un récepteur membranaire, notamment le récepteur 5-HT_{2C} de la sérotonine (le 5-HT_{2C}-R) ou celui de sous-unité B du récepteur du glutamate (GluR-B), le 5-HT_{2C}-R étant le plus préféré.

De préférence dans la méthode SSCP selon l'invention, ledit ARNm pouvant être édité est celui récepteur membranaire 5-HT_{2C}-R et le couple d'amorces est le couple suivant, de préférence marquées par des fluorophores :
PCR9 TGTCCCTAGCCATTGCTGATATGCT (SEQ ID No. 36) ; et
PCR10 GCAATCTTCATGATGGCCTTAGTCCG (SEQ ID No. 37).

Sous un autre aspect, une méthode SSCP permettant dans des conditions données d'analyse d'obtenir le profil d'édition et le taux d'édition d'un ARNm pouvant être édité, à partir d'un échantillon de tissu spécifique ou à partir d'un échantillon d'une population de cellules eucaryotes, est décrite, caractérisée en ce qu'elle comprend les étapes suivantes :
a) l'obtention du profil d'édition par la méthode SSCP ci-avant ;
b) la comparaison du profil obtenu à l'étape a) avec des profils standards correspondant à :
   - des profils caractéristiques obtenus dans ces conditions données pour chacune des formes séparées éditée (ou non) dudit ARNm ; et/ou
   - des profils caractéristiques de mélanges qualitatifs et/ou quantitatifs connus de chacune de ces formes éditées ou non, obtenus dans ces conditions données ; et/ou
   - à des profils d'édition connus dans ces mêmes conditions données, de ce même ARNm pour des patients sains ou présentant des pathologies confirmées, ceci pour des extraits d'ARNm desdits tissus spécifiques, ou encore pour ladite population de cellules eucaryotes, ces dernières ayant été de préférence cultivées dans des conditions permettant de contrôler les variations du taux d'édition dudit ARNm ;
c) la sélection du profil d'édition connu correspondant au profil d'édition obtenu à l'étape a) ; et
d) l'association du taux d'édition du profil sélectionné à l'étape c) au profil d'édition obtenu à l'étape a).

Dans ladite méthode SSCP ci-avant permettant d'obtenir à la fois le profil d'édition et le taux d'édition d'un ARNm, cette méthode incluant l'étape a) d'obtention du profil d'édition par la méthode SSCP décrite, les préférences indiquées pour la méthode SSCP (longueur des produits de PCR, ARNm d'un récepteur membranaire, notamment du 5-HT_{2C}-R ou de la sous-unité B du récepteur du glutamate (GluR-B), couple d'amorces SEQ ID No. 36 et SEQ ID No. 37 pour l'ARNm du 5-HT_{2C}-R, leur marquage) sont également revendiquées ici.

Sous encore un autre aspect, une méthode de sélection d'un composé capable de moduler l'édition d'un site d'édition situé sur un fragment d'un ARNm présent dans une cellule eucaryote, notamment de mammifère, ledit fragment d'ARNm édité ayant la séquence notée « E » et ledit fragment d'ARNm non édité ayant la séquence notée « NE », est également décrite, caractérisée en ce qu'elle comprend les étapes suivantes :
A) la mise en contact dudit composé à évaluer avec une population de cellules eucaryotes exprimant le gène dudit ARNm susceptible d'être édité ;
B) la mise en évidence de la modulation ou non de l'édition du site d'édition dudit ARNm dans ladite cellule à partir d'un échantillon d'oligonucléotides cibles dérivés dudit fragment d'ARNm et obtenus à partir d'un extrait d'acides nucléiques issu desdites cellules obtenues à l'étape A) par un procédé selon la description pour la détection et/ou la quantification d'oligonucléotides cibles dans un échantillon, ledit oligonucléotide cible étant dérivé d'un fragment d'ARNm susceptible de comporter au moins un site d'édition sur la séquence du fragment dont dérive ledit oligonucléotide cible, et dans lequel procédé au moins deux desdites sondes sont deux ADN correspondant, pour l'un à la séquence d'ADN dudit fragment « E » et, pour l'autre, à la séquence d'ADN dudit fragment « NE » ;
C) le cas échéant, en ce que l'on compare à l'étape c) du procédé ci-avant cité à l'étape B) la détection et/ou la quantification des oligonucléotides cibles capturés au niveau de chacune desdites sondes à celles obtenues à partir d'une population de cellules témoin ; et
D) la sélection de ce composé si celui-ci module l'édition dudit site d'édition.

Sous cet autre aspect, une méthode de sélection de composés capables de moduler le taux d'édition d'un fragment d'ARNm présent dans une cellule eucaryote, notamment de mammifère, est également décrite, caractérisée en ce qu'elle comprend les étapes suivantes :
A) la mise en contact dudit composé à évaluer avec une population de cellules eucaryotes exprimant ledit ARNm susceptible d'être édité ;
B) la mise en évidence de la modulation ou non du taux d'édition dudit fragment d'ARNm dans ladite cellule à partir d'un échantillon d'oligonucléotides cibles dérivés dudit fragment d'ARNm et obtenus à partir d'un extrait d'acides nucléiques issu desdites cellules obtenues à l'étape A) par un procédé selon la description pour la détection et/ou la quantification de tout oligonucléotide cible dérivé d'un fragment d'ARNm susceptible de comporter au moins un site d'édition sur la séquence du fragment dont dérive ledit oligonucléotide cible ;
C) la détermination à la fin de l'étape c) du procédé ci-avant cité à l'étape B) et pour chacune desdites sondes, du taux d'édition correspondant au rapport exprimé en pourcentage entre la quantité d'oligonucléotides capturés par une sonde et la quantité totale d'oligonucléotides capturés par l'ensemble des sondes ;
D) le cas échéant, en ce que l'on compare le taux d'édition obtenu à celui obtenu pour une population de cellules témoin ; et
E) la sélection de ce composé si celui module le taux d'édition dudit fragment d'ARNm.

L'humeur est cette disposition affective de base qui fait osciller l'être humain de la joie la plus extrême à la douleur la plus profonde, en fonction des événements et des émotions qui commandent ses réponses, en lui permettant de s'adapter, au mieux, à son environnement social, affectif, familial et professionnel. La régulation de l'humeur est un processus complexe qui fait intervenir des neuromédiateurs appartenant aux trois systèmes monoaminergiques, c'est-à-dire noradrénergique, sérotoninergique et dopaminergique. Ces neuromédiateurs agissent via des récepteurs situés dans la membrane plasmique. Lorsque ces récepteurs sont des récepteurs canaux, la liaison du ligand en module l'activité, alors que lorsqu'ils sont couplés à des effecteurs intracellulaires, la liaison des mêmes ligands conduira à une transduction d'un signal à travers la membrane plasmique. Cette transduction du signal est la conséquence d'un changement de conformation du récepteur à la suite de la liaison du ligand à la partie extracellulaire du récepteur. La spécificité de la réponse de la cellule à ces neuromédiateurs dépend, d'une part, de la nature du récepteur et donc du type d'interaction qu'il va provoquer à l'intérieur de la cellule, à la suite de son changement de conformation et, d'autre part, de la nature des cascades de réactions biochimiques ainsi induites qui seront propres au type de cellule concernée.

Il n'existe pas un récepteur responsable, à lui seul, de la régulation de l'humeur, en réponse à un neuromédiateur unique, d'autant que l'expression des gènes de ces récepteurs est interconnectée. Cependant, parmi les nouveaux antidépresseurs dits de seconde génération - ils n'appartiennent ni à la famille des antidépresseurs tricycliques, ni à celle des inhibiteurs de la mono-amine-oxydase (IMAO) - certains d'entre eux comme la fluvoxamine (Floxyfral^{®}), la fluoxétine (Prozac^{®}) et la paroxétine (Deroxat^{®}) sont considérés comme de puissants et, surtout, spécifiques inhibiteurs de la recapture de la sérotonine dans la fente synaptique (voir à ce propos, Goodnick et al. J. Psychopharmacol. 1998, 12(3) (Suppl B) S5-20). Autrement dit, les antidépresseurs de cette famille agissent en entraînant une augmentation de la concentration de sérotonine dans la fente synaptique, forçant ainsi une transduction du signal qui serait trop faible, soit par insuffisance de sérotonine, soit par insuffisance des récepteurs de la sérotononine, en nombre, ou en efficacité à transduire le signal.

La sérotonine ou 5 hydroxytryptamine (5-HT) est un neuromédiateur qui entraîne des effets physiologiques extrêmement variés, par liaison à des récepteurs de sous-types différents. La famille 5-HT₂ des récepteurs de la sérotonine appartient au grand groupe des récepteurs sept fois trans-membranaires couplés aux protéines G trimériques. Cette famille comprend trois sous-types de récepteurs (5-HT_{2A}, 5-HT_{2B} et 5-HT_{2C}) qui, par l'intermédiaire de protéines Gq, activent une phospholipase Cβ. Cette dernière, une fois activée, hydrolyse des phospholipides membranaires avec, pour conséquence, une augmentation des concentrations intra-cellulaires des inositol-phosphates (InsP) et apparition de diacylglycérol (DAG) qui reste associé à la membrane plasmique. Les récepteurs du sous-type 5-HT_{2C} (5-HT_{2C}-R) sont présents dans le système nerveux central, y compris le cortex, le striatum, l'hypothalamus, le bulbe olfactif et le plexus choroïde. Le 5-HT_{2C}-R est très certainement le ou l'un des récepteurs de la sérotonine impliqué dans la régulation de l'humeur. Il apparaît également impliqué dans la perception du désir sexuel (voir à ce propos, Lane, J., Psychopharmacol., 1997, 11(1):72-82), ainsi que dans la sensation de faim (voir à ce propos, Bickerdike et al., Diabetes, Obesity and Metabolism, 1999, 1:207-214). Sur la classification des récepteurs de la sérotonine de la famille 5-HT₂ et leur mode de transduction du signal, on se référera à Baxter et al. (Trends Pharmacol. Sci., 1995, 16(3):105-110), ainsi qu'à Roth et al. (Pharmacol Ther., 1998, 79(3):231-257) et aux articles qui y sont référencés. De même, pour la caractérisation pharmacologique des récepteurs 5-HT₂, on se réfèrera à Jerman et al. (Eur. J. Pharm., 2001, 414:23-30) et aux articles qui y sont référencés. Enfin, on trouvera la séquence complète de l'ADNc du 5-HT_{2C}-R. la structure de son gène et la description des épissages alternatifs du transcrit primaire de ce dernier, dans Xie et al. (Genomics, 1996, 35:551-561), ainsi que les séquences correspondantes dans les banques de données de Gen-Bank/EMBL sous le N° U49516 pour l'ADNc et le N° U49648 pour les séquences situées en 5' du gène.

Conformément au mode de fonctionnement des récepteurs sept fois trans-membranaires couplés aux protéines Gq trimériques dont la sous-unité αq active une phospholipase Cβ, la stimulation du 5-HT_{2C}-R par la sérotonine entraîne l'hydrolyse du phosphatidyl inositol 4,5-bisphosphate (PIP2) en inositol-1,4,5-trisphosphate (IP3) et DAG. Le DAG ainsi produit, active à son tour, une protéine kinase C qui conduira à une modification de l'expression génique, par l'intermédiaire d'une cascade de réactions biochimiques. La spécificité de cette modification de l'expression génique dépend du type de cellule concernée. L'IP3 produit par l'hydrolyse du PIP2 est une petite molécule hydrosoluble qui diffuse rapidement dans le cytosol où elle entraîne une libération de calcium en provenance du réticulum endoplasmique par interaction avec des récepteurs canaux. Cette augmentation soudaine de concentration de calcium intra-cytosolique entraîne une réponse cellulaire immédiate par modification de l'activité de protéines dont la présence entraîne la spécificité de réponse du type de cellule concernée. Deux mécanismes contrôlent la cessation de la réponse cellulaire par abaissement de la concentration du calcium intracytosolique. Le premier est le résultat du pompage du calcium hors du cytosol, principalement hors de la cellule, le second est la conséquence de l'inactivation rapide de l'IP3, principalement par sa déphosphorylation par des phosphatases spécifiques.

L'étape de déphosphorylation de l'IP3 est la cible de cet autre agent pharmacologique que sont les sels de lithium, carbonate (Téralithe^{®}) et gluconate (Neurolithium^{®}) de lithium. Utilisés dans les années 1850 dans le traitement de la goutte, puis à la fin du XIXème siècle dans le traitement de la manie, tombés dans l'oubli jusqu'au milieu du XXème siècle, les sels de lithium sont à nouveau utilisés avec un succès remarquable depuis le début des années 1970 dans le traitement des phases maniaques, en particulier dans les psychoses maniaco-dépressives où ils s'avèrent, de plus, une prophylaxie efficace des rechutes des troublés bipolaires de l'humeur. Les sels de lithium inhibent l'activité de l'inositol-1-phosphatase responsable de l'entière déphosphorylation de l'IP3 qui conduit à la formation et au recyclage de l'inositol indispensable à la synthèse du PIP2. Dans ces conditions, la diminution de la concentration de PIP2 à la face intracellulaire de la membrane plasmique n'est autre qu'une diminution du substrat de la phospholipase C-β activée, dont la conséquence est un freinage des effets de l'activation des récepteurs couplés aux protéines Gq trimériques. Autrement dit, les sels de lithium agiraient, entre autres, en entraînant une diminution du PIP2 disponible, freinant ainsi une transduction du signal qui serait trop importante, soit par excès de sérotonine, soit par excès de récepteurs de la sérotononine, en nombre ou en efficacité à transduire le signal (sur la compréhension actuelle du mode d'action des sels de lithium, voir Coyle et al., 2002, Nature Medicine, 8(6):557-558, avec les articles qui y sont référencés).

Le 5-HT_{2C}-R apparaît donc comme une des molécules-clef de la réponse à la sérotonine dans la régulation de l'humeur puisqu'il permet une réponse de la cellule dont l'amplitude dépend de son efficacité à transduire le signal. Il n'existe qu'un seul gène du 5-HT_{2C}-R, porté par le chromosome X, donc un seul allèle de ce gène chez l'homme et deux chez la femme dont l'un potentiellement inactif par inactivation de l'un des deux chromosomes X. Par conséquent, il faudrait s'attendre à ce que seuls la concentration de sérotonine extracellulaire et le nombre de récepteurs présents à la membrane à un moment donné, participent à l'efficacité de la transduction du signal en réponse à la sérotonine. En effet, si l'existence d'épissages différentiels du pré-ARNm du 5-HT_{2C}-R a bien été démontrée, il semble qu'une seule forme du 5-HT_{2C}-R puisse être sept fois trans-membranaire et active. Cependant, il existe au moins vingt-quatre sortes de récepteurs différents, produits d'expression du même gène car ils sont le résultat d'un mécanisme de régulation post-transcriptionnelle de l'expression génique appelé édition.

L'édition est le mécanisme par lequel l'information contenue dans le gène est modifiée après la transcription. On regroupe sous le terme général d'édition des ARNm, la modification de la séquence de ces ARNm qui conduit à un changement, en nature ou en nombre, des aminoacides incorporés dans la protéine au cours de la traduction, la séquence de la protéine ne pouvant plus être déduite de celle du gène qui en dirige la synthèse. L'ARN pré-messager du 5-HT_{2C}-R peut subir une modification enzymatique spécifique de certaines adénosines (A), dans la partie de ce qui deviendra l'ARNm définitif qui dirige l'incorporation des aminoacides situés dans la deuxième boucle intracellulaire du 5-HT_{2C}-R. En effet, la partie distale du cinquième exon et la partie proximale du cinquième intron du transcrit primaire sont susceptibles de former une structure en tige-boucle potentiellement reconnue par deux enzymes, ADAR1 et ADAR2 (adénosine désaminase dépendante de l'ARN double brin) qui permettent d'éditer l'ARN pré-messager, avant son épissage. Cette édition est produite par désamination de A qui sont alors transformées en inosine (I). Une fois l'épissage achevé la partie de l'ARNm qui contenait les A ayant subi l'édition, contient maintenant des I. Lors de la traduction de l'ARNm du 5-HT_{2C}-R, on pense que les I sont lues comme des G. En effet, lors de la synthèse *in vitro* de l'ADNc de l'ARNm du 5-HT_{2C}-R ayant subi la désamination des A en I, la transcriptase inverse incorpore des dC en face des I, à la place des dT qui auraient dû normalement être incorporées en face des A. Par conséquent, lors de la synthèse du second brin qui conduit à la formation de l'ADNc double brin, une dG est introduite en face de chaque dC incorporée dans le premier brin. Le séquençage de l'ADNc double brin ainsi obtenu permet de constater le remplacement des dA par des dG, en raison de la désamination initiale des A en I dans l'ARNm ayant subi l'édition. Par conséquent, l'édition de l'ARNm conduit à une modification de la signification des codons dans lesquels les A sont remplacées par des I qui seraient donc lues comme des G (plus spécifiquement, sur l'édition du 5-HT_{2C}-R humain, voir Fitzgerald et al., Neuropsychopharmacology, 1999, 21(2S), 82S-90S).

Compte tenu de ce qui a été décrit ci-dessus concernant, d'une part le mode d'action des antidépresseurs de seconde génération qui agissent en augmentant la concentration de sérotonine par inhibition de sa recapture et, d'autre part, le mode d'action des sels de lithium qui agissent sur la manie en limitant la disponibilité en PIP2, le degré (ou taux) d'édition du 5-HT_{2C}-R joue très certainement un rôle dans la régulation de l'humeur. Dans ce sens, l'étude de Niswander et al. (Neuropsychopharmacol., 2001, 24:478-491) pratiquée post-mortem, a mis en évidence que l'édition de l'ARNm du 5-HT_{2C}-R, en particulier du site A, était statistiquement plus élevée dans le cortex pré-frontal du cerveau de patients décédés par suicide que dans la même aire du cerveau de patients décédés pour d'autres raisons.

On peut également citer une autre étude effectuée par Gurevich et al. (Neuron, 2002, 34(3):349-356) qui a mis en évidence que le taux d'édition de l'ARNm du 5-HT_{2C}-R était augmenté sur le site E (appelé C' dans l'article) et diminué sur le site D dans le cortex frontal du cerveau de patients décédé par suicide, alors qu'ils souffraient de dépression majeure.

Inversement, Sodhi et al. (Mol. Psychiatry, 2001, 6(4):373-379) ont montré que le taux d'édition de l'ARNm du 5-HT_{2C}-R, était diminué dans le cortex frontal du cerveau de patients classés comme schizophrènes.

Ainsi, un récepteur non édité conduirait à un état maniaque, alors qu'un récepteur trop édité conduirait à un état dépressif. A la lumière de cette synthèse et de son interprétation faites par l'inventeur, il a été conclu qu'une modulation de taux, d'édition de l'ARNm du 5-HT_{2C}-R entraîne une modulation de l'humeur, ceci par modification de la réponse du 5-HT_{2C}-R à la stimulation par la sérotonine. Ainsi une inhibition du taux d'édition du 5-HT_{2C}-R conduira à une augmentation de la réponse à la stimulation par la sérotonine, alors qu'une stimulation ou augmentation du taux d'édition de l'ARNm du 5-HT_{2C}-R entraînera une diminution de cette même réponse à la stimulation par la sérotonine. Ceci se traduira, et de manière générale, par un effet antidépresseur pour les composés capables d'inhiber le taux d'édition de l'ARNm du 5-HT_{2C}-R et par un effet inhibiteur de la manie pour les composés capables de stimuler ou d'augmenter le taux d'édition de l'ARNm du 5-HT_{2C}-R.

Ainsi, dans un mode particulièrement préféré, une méthode de sélection d'un composé capable de moduler le taux d'édition du fragment d'ARN comprenant la séquence SEQ ID No. 33 (5'-AUA CGU AAU CCU A-3') de l'ARNm du 5-HT_{2C}-R dans une cellule eucaryote, notamment de mammifère, est décrite, caractérisée en ce qu'elle comprend les étapes suivantes :
A) la mise en contact dudit composé à évaluer avec une population de cellules eucaryotes exprimant le gène dudit ARNm ;
B) la mise en évidence de la modulation ou non du taux d'édition dudit fragment d'ARNm dans ladite cellule à partir d'un échantillon d'oligonucléotides cibles dérivés dudit fragment d'ARNm et obtenus à partir d'un extrait d'acides nucléiques issu desdites cellules obtenues à l'étape A) par un procédé selon la description pour la détection et/ou la quantification de tout oligonucléotide cible présent dans un échantillon et dérivé d'un fragment de l'ARNm du 5-HT_{2C}-R, ledit fragment comprenant la séquence SEQ ID No. 33 (5'-AUA CGU AAU CCU A-3') édité ou non édité ;
C) la détermination à la fin de l'étape c) du procédé ci-avant cité à l'étape B) et pour chacune desdites sondes, du taux d'édition correspondant au rapport exprimé en pourcentage entre la quantité d'oligonucléotides capturés par une sonde et la quantité totale d'oligonucléotides capturés par l'ensemble des sondes ;
D) le cas échéant, en ce que l'on compare le taux d'édition obtenu à celui obtenu pour une population de cellules témoin ; et
E) la sélection de ce composé si celui-ci module le taux d'édition dudit fragment d'ARNm.

Sous un autre aspect, une méthode de sélection d'un composé capable de moduler le taux et/ou le profil d'édition d'un ARNm susceptible d'être édité dans un tissu spécifique ou une population de cellules eucaryotes exprimant le gène dudit ARNm, notamment de mammifère tel que l'homme ou la souris, est décrite, caractérisée en ce qu'elle comprend les étapes suivantes :
a) la mise en contact, *in vivo* ou *in cellulo*, dudit composé à évaluer avec ledit tissu spécifique ou ladite population de cellules eucaryotes ;
b) l'obtention du profil d'édition par la méthode SSCP selon la description dans des conditions données d'analyse ;
c) la comparaison du profil obtenu à l'étape b) avec :
   - soit des profils standards correspondant à des profils d'édition connus de ce même ARNm, pour un même tissu spécifique ou une même population de cellules eucaryotes, ce dans les mêmes conditions d'analyse données, ou
   - soit à un profil d'édition fait en parallèle et obtenu pour un même tissu spécifique témoin ou une même population de cellules eucaryotes témoins n'ayant pas été mis en contact avec le composé à évaluer ; et
d) la sélection dudit composé à évaluer si les profils d'édition comparés à l'étape c) sont significativement différents entre eux.

Dans ladite méthode ci-avant de sélection d'un composé capable de moduler le taux et/ou le profil d'édition d'un ARNm et incluant, à l'étape b) l'obtention du profil d'édition par la méthode SSCP selon la description, les préférences indiquées pour la méthode SSCP (longueur des produits de PCR, ARNm d'un récepteur membranaire, notamment du 5-HT_{2C}-R ou de la sous-unité B du récepteur du glutamate (GluR-B), couple d'amorces SEQ ID No. 36 et SEQ ID No. 37 pour l'ARNm du 5-HT_{2C}-R, leur marquage) sont également revendiquées ici.

Sous un autre aspect, une méthode de sélection d'un composé capable de prévenir et/ou traiter chez un patient une pathologie associée, au moins en partie, à l'édition d'un ARNm susceptible d'être édité, est décrite, caractérisée en ce qu'elle comprend les étapes suivantes :
a) la mise en contact, *in vivo* ou *in cellulo*, dudit composé à évaluer avec un tissu spécifique ou une population de cellules eucaryotes exprimant le gène dudit ARNm susceptible d'être édité, ledit tissu spécifique ou ladite population de cellules eucaryotes présentant avant la mise en contact du composé à tester un profil d'édition dudit ARNm caractéristique de la pathologie associée et ce, dans des conditions données d'analyse ;
b) l'obtention du profil d'édition par la méthode SSCP selon la description dans ces conditions données d'analyse ;
c) la comparaison du profil obtenu à l'étape b) avec :
   α) un profil standard correspondant à un profil d'édition connu de ce même ARNm, pour un même tissu spécifique ou pour une même population de cellules, ce dans les mêmes conditions données d'analyse, ce profil d'édition étant représentatif d'un patient sain ou ne présentant pas ladite pathologie associée ; et, le cas échéant avec :
   β) un profil d'édition obtenu pour un même tissu spécifique témoin ou pour une même population de cellules témoins n'ayant pas été mis en contact avec le composé à évaluer, ce dans les mêmes conditions données d'analyse ; et
d) la sélection dudit composé à évaluer si les profils d'édition comparés à l'étape c) montrent que celui obtenu à l'étape b) est significativement identique à celui de l'étape c)α), et, le cas échéant confirmé cette sélection si celui obtenu à l'étape b) est significativement différent de celui de l'étape c)β).

Dans ladite méthode ci-avant de sélection on d'un composé capable de prévenir et/ou traiter chez un patient une pathologie associée à l'édition d'un ARNm et incluant, à l'étape b) l'obtention du profil d'édition par la méthode SSCP selon la description, les préférences indiquées pour la méthode SSCP (longueur des produits de PCR, ARNm d'un récepteur membranaire, notamment du 5-HT_{2C}-R ou de la sous-unité B du récepteur du glutamate (GluR-B), couple d'amorces SEQ ID No. 36 et SEQ ID No. 37 pour l'ARNm du 5-HT_{2C}-R. leur marquage) sont également revendiquées ici.

Sous un aspect particulier, est décrite une méthode de sélection d'un composé capable de prévenir et/ou traiter chez un patient une pathologie associée, au moins en partie, à l'édition ou non d'un ARNm susceptible d'être édité avec un même mécanisme thérapeutique ou efficacité qu'un composé connu pour moduler le profil d'édition dudit ARN et connu pour prévenir et/ou traiter chez un patient la même pathologie associée, caractérisée en ce qu'elle comprend les étapes suivantes :
a) la mise en contact, *in vivo* ou *in cellulo*, dudit composé à évaluer avec un tissu spécifique ou une population de cellules eucaryotes exprimant le gène dudit ARNm susceptible d'être édité, ledit tissu spécifique ou ladite population de cellules eucaryotes présentant avant la mise en contact du composé à tester un profil d'édition dudit ARNm caractéristique de la pathologie associée et ce, dans des conditions données d'analyse ;
b) l'obtention du profil d'édition par la méthode SSCP selon la description dans ces conditions données d'analyse
c) la comparaison du profil obtenu à l'étape b) avec :
   α) un profil standard correspondant à un profil d'édition connu de ce même ARNm, pour un même tissu spécifique ou pour une même population de cellules ayant été mis en contact, *in vivo* ou *in cellulo*, avec ledit composé connu pour moduler le profil d'édition dudit ARN dans les mêmes conditions données d'analyse et connu pour prévenir et/ou traiter chez un patient la même pathologie associée ; et, le cas échéant avec :
   β) un profil d'édition obtenu pour un même tissu spécifique témoin ou pour une même population de cellules témoins n'ayant pas été mis en contact avec le composé à évaluer, ce dans les mêmes conditions données d'analyse ; et
d) la sélection dudit composé à évaluer si les profils d'édition comparés à l'étape c) montrent que celui obtenu à l'étape b) est significativement identique à celui de l'étape c)α), et, le cas échéant confirmé cette sélection si celui obtenu à l'étape b) est significativement différent de celui de l'étape c)β).

Dans ladite méthode ci-avant de sélection d'un composé capable de prévenir et/ou traiter chez un patient une pathologie associée, au moins en partie, à l'édition ou non d'un ARNm susceptible d'être édité avec un même mécanisme thérapeutique ou efficacité qu'un composé connu et incluant, à l'étape b) l'obtention du profile d'édition par la méthode SSCP selon la description, les préférences indiquées pour la méthode SSCP (longueur des produits de PCR, ARNm d'un récepteur membranaire, notamment du 5-HT_{2C}-R ou de la sous-unité B du récepteur du glutamate (GluR-B), couple d'amorces SEQ ID No. 36 et SEQ ID No. 37 pour l'ARNm du 5-HT_{2C}-R, leur marquage) sont également revendiquées ici.

Sous encore un autre aspect, est décrite une méthode de diagnostic, le cas échéant prédictif, de maladie associée, au moins en partie, à un ARNm susceptible d'être édité, à partir d'un échantillon de tissu ou de cellules prélevé chez un patient à tester, caractérisée en ce qu'elle comprend les étapes suivantes :
a) l'obtention du profil d'édition dudit ARNm par la méthode SSCP selon la description dans des conditions d'analyse données ;
b) la comparaison du profil obtenu à l'étape a) avec des profils standards correspondant à des profils d'édition connus de ce même ARNm pour des patients sains ou présentant des pathologies confirmées, ceci pour des extraits d'ARNm de même tissu ou de même cellule, dans ces mêmes conditions données, ou encore pour des cellules issues de lignées cellulaires ; et
c) la sélection du profil d'édition connu correspondant au profil d'édition obtenu à l'étape a) ; et
d) l'association du diagnostic attaché au profil sélectionné à l'étape c) au patient testé.

Dans ladite méthode ci-avant de diagnostic de maladie associée, au moins en partie, à un ARNm et incluant, à l'étape a) l'obtention du profil d'édition par la méthode SSCP selon la description, les préférences indiquées pour la méthode SSCP (longueur des produits de PCR, ARNm d'un récepteur membranaire, notamment du 5-HT_{2C}-R ou de la sous-unité B du récepteur du glutamate (GluR-B), couple d'amorces SEQ ID No. 36 et SEQ ID No. 37 pour l'ARNm du 5-HT_{2C}-R, leur marquage) sont également revendiquées ici.

Sous un autre aspect particulier, est décrite une première méthode de sélection d'un composé capable de moduler le taux d'édition du fragment d'ARN comprenant la séquence SEQ ID No. 33 (5'-AUA CGU AAU CCU A-3') de l'ARNm du 5-HT_{2C}-R, ou une deuxième méthode de sélection d'un composé capable de moduler le taux et/ou le profil d'édition de l'ARNm du 5-HT_{2C}-R incluant à l'étape b) ladite méthode SSCP d'obtention du profil d'édition, caractérisée en ce qu'à l'étape E) de la première méthode citée, ou à l'étape d) de la deuxième méthode citée, on sélectionne le composé si celui-ci ne module pas en outre le taux ou le profil d'édition de l'ARNm de la sous-unité B du récepteur du glutamate (GluR-B).

Sous un autre aspect particulier, est décrite une première méthode de sélection d'un composé capable de moduler le taux d'édition du fragment d'ARN comprenant la séquence SEQ ID No. 33 (5'-AUA CGU AAU CCU A-3') de l'ARNm du 5-HT_{2C}-R, ou une deuxième méthode de sélection d'un composé capable de moduler le taux et/ou le profil d'édition de l'ARNm du 5-HT_{2C}-R incluant à l'étape b) ladite méthode SSCP d'obtention du profil d'édition, caractérisée en ce qu'à l'étape E) de la première méthode citée, ou à l'étape d) de la deuxième méthode citée, on sélectionne le composé si celui-ci diminue le taux d'édition d'au moins un site d'édition dudit fragment d'ARN, site d'édition qui lorsqu'il est édité modifie la séquence des acides aminés de la deuxième boucle intracellulaire du 5-HT_{2C}-R, notamment si cette diminution du taux d'édition augmente la capacité des cellules exprimant le gène du 5-HT_{2C}-R à répondre à une stimulation par la sérotonine.

Sous un autre aspect particulier, est décrite une première méthode de sélection d'un composé capable de moduler le taux d'édition du fragment d'ARN comprenant la séquence SEQ ID No. 33 (5'-AUA CGU AAU CCU A-3') de l'ARNm du 5-HT_{2C}-R, ou une deuxième méthode de sélection d'un composé capable de moduler le taux et/ou le profil d'édition de l'ARNm du 5-HT_{2C}-R incluant à l'étape b) ladite méthode SSCP d'obtention du profil d'édition, caractérisée en ce qu'à l'étape E) de la première méthode citée, ou à l'étape d) de la deuxième méthode citée, on sélectionne le composé si celui-ci augmente le taux d'édition d'au moins un site d'édition dudit fragment d'ARN, site d'édition qui lorsqu'il est édité modifie la séquence des acides aminés de la deuxième boucle intracellulaire du 5-HT_{2C}-R, notamment si cette augmentation du taux d'édition diminue la capacité des cellules exprimant le gène du 5-HT_{2C}-R à répondre à une stimulation par la sérotonine.

Sous encore un autre aspect, est décrite l'utilisation d'un composé capable de moduler le taux d'édition de l'ARNm du 5-HT_{2C}-R, ledit composé ayant été sélectionné ou étant susceptible d'être sélectionné par un procédé de sélection d'un composé capable de moduler le taux d'édition de l'ARNm du 5-HT_{2C}-R dans une cellule eucaryote, notamment de mammifère, pour la préparation d'une composition pharmaceutique destinée à moduler l'humeur chez un patient nécessitant un tel traitement, notamment pour la préparation d'une composition pharmaceutique destinée soit au traitement de la dépression si ce composé sélectionné diminue le taux d'édition dudit site d'édition, soit au traitement de la manie ou de certaines formes de schizophrénie si ce composé sélectionné augmente le taux d'édition dudit site d'édition.

Sous encore un autre aspect, est décrit un composé ayant une activité modulatrice du taux d'édition du fragment d'ARN comprenant la séquence SEQ ID No. 33 (5'-AUA CGU AAU CCU A-3') de l'ARNm du 5-HT_{2C}-R pour le traitement de l'humeur.

Pour les troubles de l'humeur pouvant être améliorés par des médicaments modulant l'édition du 5-HT_{2C}-R, on pourra se référer à l'ouvrage "Diagnostic and Statistical Manual of Mental Disorders, fourth edition, text revision, published by the American Psychiatric Association, Washington DC, 2000, DSM-IV^{™}.

On peut citer de manière particulière pour les dépressions :
- la dépression majeure caractérisée par un ou plusieurs épisodes dépressifs (c'est-à-dire au moins 2 semaines d'humeur dépressive avec perte d'intérêt accompagnée par au moins 4 symptômes additionnels de la dépression) (voir DSM-IV p349-369) ;
- la dépression bipolaire :
- bipolaire I : caractérisée par un ou plusieurs épisodes maniaques ou mixtes, généralement accompagnés d'épisodes de dépression majeures (voir DSM-IV p382),
- bipolaire II : caractérisée par un ou plusieurs épisodes de dépression majeure accompagnés par au moins un épisode hypomaniaque (voir DSM-IV p392), et
- les troubles cyclothymiques : caractérisés par au moins deux semaines de nombreuses périodes de symptômes hypomaniaques et de symptômes dépressifs (voir DSM-IV p398) ;
- la mélancolie : les états mélancoliques se rencontrent chez les dépressifs majeurs ainsi que chez les dépressifs bipolaires. Cet état est caractérisé par une perte d'intérêt et/ou de plaisir dans toutes les activités, ainsi que par une diminution de la réactivité aux stimuli déclenchant habituellement le plaisir (voir DSM-IV p419) ; et
- la schizophrénie : caractérisée par des troubles durant au moins six mois et incluant au moins un mois de symptômes positifs (délusions, hallucination, désorganisation du langage) et symptômes négatifs (de type dépressifs) (voir DSM-IV p298).

Sous cet autre aspect, est décrit un composé capable de diminuer le taux d'édition d'au moins un site d'édition du fragment d'ARN comprenant la séquence SEQ ID No. 33 (5'-AUA CGU AAU CCU A-3') de l'ARNm du 5-HT_{2C}-R, site d'édition qui lorsqu'il est édité modifie la séquence des acides aminés de la deuxième boucle intracellulaire du 5-HT_{2C}-R, pour le traitement de la dépression, notamment si cette diminution du taux d'édition augmente la capacité des cellules exprimant le gène du 5-HT_{2C}-R à répondre à une stimulation par la sérotonine.

Sous cet autre aspect, est décrit un composé capable d'augmenter le taux d'édition d'un site d'édition du fragment d'ARN comprenant la séquence SEQ ID No. 33 (5'-AUA CGU AAU CCU A-3') de l'ARNm du 5-HT_{2C}-R, site d'édition qui lorsqu'il est édité modifie la séquence des acides aminés de la deuxième boucle intracellulaire du 5-HT_{2C}-R, pour le traitement de la manie ou de certaines formes de schizophrénie, notamment si cette augmentation du taux d'édition diminue la capacité des cellules exprimant le gène 5-HT_{2C}-R à répondre à une stimulation par la sérotonine.

Les exemples suivants ainsi que les figures et les légendes ci-après ont été choisis pour fournir à l'homme de l'art une description complète afin de pouvoir réaliser et utiliser la présente invention. Ces exemples ne sont pas destinés à limiter la portée de ce que l'inventeur considère comme son invention, ni destinés à montrer que seules les expériences ci-après ont été effectuées.

### Légendes des figures

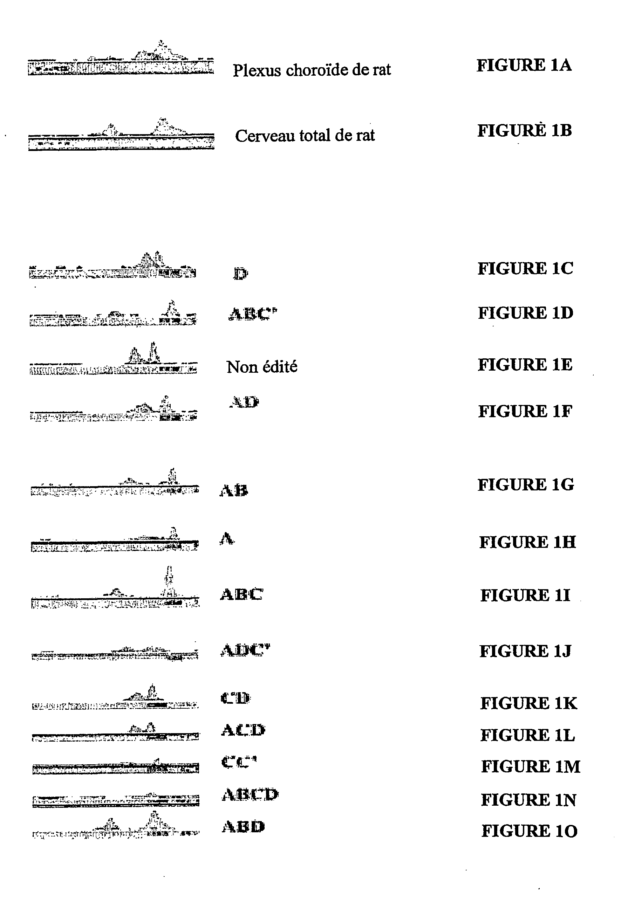

Figures 1A à 1O : Exemples de profils analytiques de l'édition du récepteur 5-HT_{2C} obtenus par la méthode de SSCP décrite plus haut. Dans les conditions de cet exemple (amplification d'un fragment de 250 paires de bases et analyse par électrophorèse capillaire) les profils d'édition obtenus à partir d'ARN provenant de plexus choroïde de rat (figure 1A) et de cerveau total de rat (figure 1B) sont caractéristiques de chacune de ces structures. L'importance relative de chacune des mutations peut être déterminée à partir des profils standards d'édition pour chacune des formes séparées d'édition qui sont caractéristiques pour chaque forme correspondant à une combinaison mutante donnée du récepteur 5-HT_{2C} sur un ou plusieurs des 5 sites d'édition (A, B, C, D et C') amplifiés et analysés dans les mêmes conditions (figures 1C à 1O). Les profils d'édition de toutes les formes séparées d'édition ont été obtenus mais ne sont pas tous représentés ici.

### EXEMPLE 1 : Méthode permettant de déterminer, par hybridation spécifique et en une seule étape, le pourcentage de chacune des trente-deux formes de l'ARNm du 5-HT_{2C}-R.

La méthode décrite ci-après permet de déterminer, par hybridation spécifique et en une seule étape, le pourcentage de chacune des trente-deux formes de l'ARNm du 5-HT_{2C}-R même si l'amplification de l'information contenue dans ces ARNm a été nécessaire.

Après édition des ARNm, les A sont remplacées par des 1 qui sont, à leur tour, remplacées par des dG dans l'ADNc. Autrement dit, plus le nombre de A transformées en I au cours de l'édition de l'ARNm initial est grand, plus la température nécessaire à une hybridation spécifique est élevée. Le remplacement des dG par des dl permet d'effectuer les hybridations à une température unique selon la stratégie décrite ci-dessous.

Si nécessaire, en raison d'une quantité d'ARNm de départ trop faible, l'information amplifiée *in vitro* peut l'être sous forme d'ARN simple brin, de façon linéaire par transcription *in vitro* (IVT pour "in vitro transcription") ; elle peut l'être aussi sous forme d'ADN double brin par RT-PCR après transcription inverse (RT pour "reverse transcription") de l'ARNm suivie d'une amplification exponentielle par PCR. Que l'information soit amplifiée sous la forme finale d'un ARN simple brin ou sous celle d'un ADN double brin, pose le problème de l'identification des I puisque, dans tous les cas, au cours de la synthèse de l'ADNc par la transcriptase inverse, une dC est incorporée en face de chaque I et, lors de la synthèse du brin complémentaire, une dG est ensuite incorporée en face de chaque dC. Ceci revient à dire que si les séquences qui sont hybridées aux différentes sondes sont celles complémentaires des ARNm initiaux, elles contiennent des dT ou des U en face de chaque A non éditée et des dC ou des C en face de chaque I, selon que les séquences ont été obtenues sous forme d'ADN par RT-PCR ou sous forme d'ARN par IVT.

Dans le cas où l'information contenue dans les ARNm de départ est amplifiée par IVT du brin sens, les molécules d'ARN synthétisées présentent une séquence identique à celle des ARNm mais dont les A, éditées en I, sont remplacées par des G. Inversement, l'IVT du brin anti-sens permet de synthétiser des molécules d'ARN dont la séquence est complémentaire de celle des ARNm de départ mais avec des C qui remplacent les U en face des sites édités de A en I.

Par conséquent, pour que les températures d'hybridation soient identiques pour toutes les sondes, les ADN simple brin déposés sous forme de sondes sur le support sont de séquence identique à celles des ARNm initiaux mais dans lesquelles des dA remplacent les A non éditées et des dl remplacent des I provenant de l'édition de ces A en I. Ainsi, que les ARN initiaux aient été amplifiés par RT-PCR sous forme d'ADN double brin ou par IVT sous forme d'ARN antisens des ARNm initiaux, il y aura toujours formation de deux liaisons hydrogènes entre dA et dT ou U d'une part et entre dl et dC ou C d'autre part.

Les séquences des trente-deux sondes nécessaires à l'identification des différentes formes, éditées ou non, de l'ARNm du 5-HT_{2C}-R sont rapportées ci-dessous.

Les Tm ayant été mesurées individuellement pour chacune des sondes, toutes les conditions autres que la température étant identiques par ailleurs, la température d'hybridation pour l'ensemble des sondes est choisie entre les deux Tm extrêmes mesurées, ici pour la sonde de séquence SEQ ID No. 1 et la sonde de séquence SEQ ID No. 32.

Les conditions autres que la Tm auront été définies de façon à ce que l'écart entre les deux Tm extrêmes mesurées soient le plus faible possible.

Les différentes formes initiales de l'ARNm du 5-HT_{2C}-R sont quantifiées par mesure soit de la radioactivité, soit de l'intensité de fluorescence des trente-deux séquences appariées. La somme des trente-deux valeurs mesurées étant directement proportionnelle à la somme des différentes formes initiales de l'ARNm du 5-HT_{2C}-R, le pourcentage de chacune des formes de cet ARNm peut être déduit en divisant la valeur mesurée individuellement pour chaque forme par la somme des valeurs des trente-deux formes.

| Sites d'édition | | A B EC D | |
|---|---|---|---|
| 1 | 5-HT_{2C}R-0 I-N-I | d(CAATACGTAATCCTATT) | SEQ ID No. 1 |
| 2 | 5-HT_{2C} R-A V-N-I | d(CAITACGTAATCCTATT) | SEQ ID No. 2 |
| 3 | 5-HT_{2C} R-B M-N-I | d(CAATICGTAATCCTATT) | SEQ ID No. 3 |
| 4 | 5-HT_{2C} R-C I-S-I | d(CAATACGTAITCCTATT) | SEQ ID No. 4 |
| 5 | 5-HT_{2C} R-D I-N-V | d(CAATACGTAATCCTITT) | SEQ ID No. 5 |
| 6 | 5-HT_{2C} R-E I-D-I | d(CAATACGTIATCCTATT) | SEQ ID No. 6 |
| 7 | 5-HT_{2C} R-AB V-N-I | d(CAITICGTAATCCTATT) | SEQ ID No. 7 |
| 8 | 5-HT_{2C} R-AC V-S-I | d(CAITACGTAITCCTATT) | SEQ ID No. 8 |
| 9 | 5-HT_{2C} R-AD V-N-V | d(CAITACGTAATCCTITT) | SEQ ID No. 9 |
| 10 | 5-HT_{2C} R-AE V-D-I | d(CAITACGTIATCCTATT) | SEQ ID No. 10 |
| 11 | 5-HT_{2C} R-BC M-S-I | d(CAATICGTAITCCTATT) | SEQ ID No. 11 |
| 12 | 5-HT_{2C} R-BD M-N-V | d(CAATICGTAATCCTITT) | SEQ ID No. 12 |
| 13 | 5-HT_{2C} R-BE M-D-I | d(CAATICGTIATCCTATT) | SEQ ID No. 13 |
| 14 | 5-HT_{2C} R-CD I-S-V | d(CAATACGTAITCCTITT) | SEQ ID No. 14 |
| 15 | 5-HT_{2C} R-CE I-G-I | d(CAATACGTIITCCTATT) | SEQ ID No. 15 |
| 16 | 5-HT_{2C} R-DE I-D-V | d(CAATACGTIATCCTITT) | SEQ ID No. 16 |
| 17 | 5-HT_{2C} R-ABC V-S-I | d(CAITICGTAITCCTATT) | SEQ ID No. 17 |
| 18 | 5-HT_{2C} R-ABD V-N-V | d(CAITICGTAATCCTITT) | SEQ ID No. 18 |
| 19 | 5-HT_{2C} R-ABE V-D-I | d(CAITICGTIATCCTATT) | SEQ ID No. 19 |
| 20 | 5-HT_{2C} R-ACD V-S-V | d(CAITACGTAITCCTITT) | SEQ ID No. 20 |
| 21 | 5-HT_{2C} R-ACE V-G-I | d(CAITACGTIITCCTATT) | SEQ ID No. 21 |
| 22 | 5-HT_{2C} R-ADE V-D-V | d(CAITACGTIATCCTITT) | SEQ ID No. 22 |
| 23 | 5-HT_{2C} R-BCD M-S-V | d(CAATICGTAITCCTITT) | SEQ ID No. 23 |
| 24 | 5-HT_{2C} R-BCE M-G-I | d(CAATICGTIITCCTATT) | SEQ ID No. 24 |
| 25 | 5-HT_{2C} R-BDE M-D-V | d(CAATICGTIATCCTITT) | SEQ ID No. 25 |
| 26 | 5-HT_{2C} R-CDE I-G-V | d(CAATACGTIITCCTITT) | SEQ ID No. 26 |
| 27 | 5-HT_{2C} R-ABCDV-S-V | d(CAITICGTAITCCTITT) | SEQ ID No. 27 |

| | | | |
|---|---|---|---|
| 28 | 5-HT_{2C} R-ABCE V-G-I | d(CAITICGTIITCCTATT) | SEQ ID No. 28 |
| 29 | 5-HT_{2C} R-ABDE V-D-V | d(CAITICGTIATCCTITT) | SEQ ID No. 29 |
| 30 | 5-HT_{2C} R-ACDE V-G-V | d(CAITACGTIITCCTITT) | SEQ ID No. 30 |
| 31 | 5-HT_{2C} R-BCDE M-G-V | d(CAATICGTIITCCTITT) | SEQ ID No. 31 |
| 32 | 5-HT_{2C} R-ABCDE V-G-V | d(CAITICGTIITCCTITT) | SEQ ID No. 32 |

Les Tm ayant été mesurées individuellement pour chacune des sondes, toutes les conditions autres que la température étant identique par ailleurs, les inventeurs ont ainsi mis en évidence qu'il était possible de réduire de manière très significative l'écart obtenu entre les deux Tm extrêmes mesurées (ΔTm ₑₓₜ) pour la sonde de séquence SEQ ID No. 1 et la sonde de séquence SEQ ID No. 32, par rapport à l'écart obtenu entre les deux Tm extrêmes mesurées pour les sondes normales (jeu de sondes dans lequel les dl sont remplacés par des dG).

### Résultats

- pour le jeu de sondes de séquences SEQ ID Nos. 1 à 32 de l'invention :
   ΔTm ₑₓₜ(en °C) = 61, 1 ± 0,13 - 54, 13 ± 0,44 soit environ 6,97°C ;
- pour le jeu de sondes normales (sans dl) :
   ΔTm ₑₓₜ (en °C) = 74, 1 ± 0,2 - 61, 1 ± 0,13 soit environ 13,00°C.

### Spécificité du jeu de sondes

Les inventeurs ont mis également en évidence qu'à la température d'hybridation qui sera choisie pour l'ensemble des sondes décrites (entre 61, 1 et 54, 13°C, de préférence autour de la valeur moyenne de 57, 6°C, la sonde SEQ ID No. 32 n'hybride pas avec un oligonucléotide complémentaire de cette sonde à l'exception de la base située en complément du dl situé en position 15 de la sonde SEQ ID No. 32 (Tm de 49, 4°C).

### EXEMPLE 2 : Exemple de réalisation

L'exemple de réalisation développé ci-dessous concerne l'édition des ARNm avec, pour application, celle des ARNm de l'un des récepteurs de la sérotonine. Cet exemple montre qu'il est possible d'effectuer une hybridation dans des conditions de température unique, même lorsque le pourcentage de guanines et cytosines initialement appariées passe de moins de 30 % à près de 60 %, dans une séquence courte.

Cinq adénosines sont potentiellement éditables dans trois codons différents ce qui conduit à 2⁵ = 32 combinaisons différentes de séquences d'ARNm et 3x4x2 = 24 combinaisons différentes de séquences en aminoacides (voir schéma ci-dessous). La désamination des adénosines aux cinq sites éditables, notés de A à E (ou encore A, B, C, D et C'), conduit au remplacement de trois aminoacides de la deuxième boucle intracellulaire du 5-HT_{2C}-R. Le 5-HT_{2C}-R synthétisé par traduction de l'ARNm non édité (SEQ ID No. 34) est constitué des aminoacides I-N-I (isoleucine - asparagine - isoleucine) dans la deuxième boucle intracellulaire, tandis que celui synthétisé par traduction de l'ARNm complètement édité (SEQ ID No. 35) ou édité simultanément aux sites A, E, C et D est constitué des aminoacides V-G-V (valine - glycine - valine), les codons IUI ou IUA spécifiant tous les deux l'incorporation d'une valine, si I est effectivement lue comme G. Les codons AUI, AIU et IAU spécifiant respectivement l'incorporation de M (méthionine), S (sérine) et D (acide aspartique), vingt-quatre combinaisons d'aminoacides sont effectivement possibles dans la deuxième boucle intracellulaire du 5-HT_{2C}-R, pour trente-deux combinaisons de séquences différentes de son ARNm.

A B EC D Sites d'édition
5'-CA AUA CGU AAU CCU AUU-3' ARNm du 5-HT2C-R non édité
I R N P I Aminoacides du 5 HT2c R (ARNm non édité)

5'-CA IUI CGU IIU CCU IUU-3' ARNm du 5-HT2C-R tout édité
V G V Aminoacides du 5-HT2C-R (ARNm tout édité)

IUA Édition du site A
V Aminoacide du 5-HT2C-R (ARNm édité au site A)

AUI AIU Édition des sites B et C
M S Aminoacides du 5-HT2C-R
(ARNm édité aux sites B et C)

IAU Édition du site E

D		Aminoacide du 5-HT2C-R (ARNm édité au site E)

La deuxième boucle intracellulaire du 5-HT_{2C}-R est impliquée dans le couplage du récepteur aux protéines Gq trimériques. Un 5-HT_{2C}-R (I-N-I) synthétisé par traduction de l'ARNm non édité présente une activité constitutive de couplage, donc une activation permanente de la phospholipase Cβ, tandis qu'un 5-HT_{2C}-R (V-G-V) synthétisé par traduction d'un ARNm complètement édité répond moins bien à la stimulation par la sérotonine par défaut de couplage aux protéines Gq trimériques. Autrement dit, selon son degré d'édition, le 5-HT_{2C}-R présentera une aptitude plus ou moins grande à répondre à la stimulation par la sérotonine.

Pouvoir mesurer le pourcentage représenté par chacune des trente-deux formes possibles d'ARNm, revient à prédire le pourcentage de chacune des vingt-quatre formes du 5-HT_{2C}-R et, par conséquent, à pouvoir estimer indirectement l'efficacité de réponse du 5-HT_{2C}-R à la stimulation par la sérotonine. Deux méthodes ont été utilisées pour estimer les pourcentages des formes différentes d'ARNm du 5-HT_{2C}-R. Ces deux méthodes ne donnent pas des renseignements équivalents.

La première méthode, techniquement la plus lourde et la plus coûteuse, permet d'estimer statistiquement le pourcentage de chacune des trente-deux formes de l'ARNm du 5-HT_{2C}-R. Elle consiste à synthétiser simultanément tous les ADNc des ARNm du 5-HT_{2C}-R grâce à des amorces spécifiques communes aux différentes formes d'ARNm, puis à insérer tous ces ADNc dans des plasmides qui sont ensuite transfectés dans des bactéries. Après transformation et sélection, ces dernières sont ensuite clonées. Ces bactéries clonées sont alors amplifiées, puis l'ADN plasmidique en est extrait, purifié et les ADNc insérés sont alors séquencés. Si le nombre d'ADNc clonés et séquencés est suffisant pour être statistiquement représentatif de la population initiale d'ADNc donc d'ARNm, le pourcentage de chacune des formes initiales d'ARNm du 5-HT_{2C}-R pourra alors être déduit. Si ce nombre est insuffisant, seules les formes majoritaires de l'ARNm du 5-HT_{2C}-R seront statistiquement représentées.

La seconde méthode est plus rapide à mettre en oeuvre mais elle ne permet pas d'estimer le pourcentage de chacune des trente-deux formes de l'ARNm du 5-HT_{2C}-R. Elle permet, en revanche, de déterminer le pourcentage de chaque A éditée en I, à chacun des cinq sites éditables. Pour cela, après synthèse des ADNc comme dans le cas précédent, il est procédé à une extension d'amorce, en présence de trois des quatre désoxyribonucléosides triphosphates habituels et d'un didésoxyribonucléoside triphosphate qui termine l'extension de l'amorce en face du désoxyribonucléoside dont on veut déterminer la nature. Par exemple les trois désoxyribonucléosides triphosphates peuvent être dATP, dGTP et dTTP, alors que le didésoxyribonucléoside triphosphate est le ddCTP (didésoxycytidine triphosphate) qui, en s'incorporant en face de la première dG rencontrée, arrête l'extension de l'amorce là où se trouvait une I dans l'ARNm qui a servi de matrice à la synthèse de l'ADNc. Dans le cas où l'adénosine initiale n'aurait pas été éditée et donc remplacée par une dG dans l'ADNc, l'amorce est allongée jusqu'à la première dG qui suit la dA provenant de l'adénosine non éditée. Dans ces conditions, les amorces doivent être judicieusement choisies, complémentaires de l'un ou l'autre des deux brins de l'ADNc et doivent, en outre, avoir été marquées au ³²P en 5', pour être visualisées par autoradiographie après séparation par électrophorèse en gel de polyacrylamide. La mesure de la radioactivité contenue dans les différents fragments séparés permet d'estimer le pourcentage de A édités à chacun des cinq sites éditables. Il faut, au moins, autant d'amorces qu'il y a de sites éditables.

### EXEMPLE 3 : Identification et quantification des différentes formes (éditées ou non) et détermination du profil d'édition des ARNm par SSCP (Single Strand Conformational Polymorphism) des ADN complémentaires

### I) PROTOCOLE

### 1 - Extraction des ARN totaux :

Pour extraire les ARN de tissus congelés dans la solution RNA Later (annexe C), ou fraîchement disséqués, ces tissus sont broyés dans 350 µl de la solution RA1 du kit d'extraction NucleoSpin RNA II (Annexe B1), additionnée de 3,5 µl de β-mercapto-éthanol (Annexe C). Le lysat est filtré par centrifugation sur une colonne de filtration (Annexe B1) à 11000 x g pendant 1 minute. Après homogénéisation du filtrat avec 350 µl d'une solution d'éthanol 70°, l'ensemble est centrifugé sur une colonne de silice Nucleospin RNAII (annexe B1) pendant 30 secondes à 8 000 x g. La colonne est alors lavée par 350 µl de la solution MDB (solution tampon de dessalage de membrane) (MDB pour « Membrane desalting Buffer ») (Annexe B1) et centrifugation pendant 30 secondes à 11 000 x g. Afin d'éliminer l'ADN génomique contaminant éventuel, on place sur la colonne 10 µl de la solution de DNase1 (Annexe B1) pendant 30 minutes à température ambiante. Après digestion complète de l'ADN, la DNase I est éliminée par un lavage avec 200 µl de la solution RA2 (Annexe B1) puis un autre avec 600 µl de la solution RA3 (Annexe B1), suivis chacun par une centrifugation de 30 secondes à 8 000 x g. L'ARN fixé sur la colonne est lavé une dernière fois avec 250 µl de la solution RA3 (Annexe B1) et finalement séché par une centrifugation de 2 minutes à 11000 x g. L'élution de l'ARN est effectuée avec 60 µl d'eau sans RNAse (Annexe B1), après centrifugation pendant 1 minute à 11 000 x g.

### 2 - Transcription inverse :

Pour cette étape, on utilise les kit et protocole ThermoScript RT-PCR system (Annexe B2). Pour chaque échantillon d'ARN, on mélange 500 ng d'ARN total avec 1 µl de poly-(dT)20 (50 µM ; Annexe B2), complété éventuellement avec de l'eau sans RNase (Annexe B2) pour un volume final de 10 µl. Ce volume est incubé pendant 5 minutes à 65°C, puis immédiatement refroidi dans la glace. On ajoute ensuite 4 µl du tampon de synthèse de l'ADNc (5 x concentré ; Annexe B2), 1 µl de la solution de DTT (DTT pour Di-Thiothréitol) (0,1 M ; Annexe B2), 1 µl de RNase OUT (40 U/µl ; Annexe B2), 1 µl d'eau sans RNase (Annexe B2), 2 µl d'un mélange de dNTP (dNTP : désoxyribonucléotide triphosphate) (10 mM chacun ; Annexe B2) et 1 µl de l'enzyme thermoScript RT (15 U/µl ; Annexe B2), et l'ensemble est incubé à 50°C pendant 60 minutes. La réaction est alors arrêtée en chauffant le mélange réactionnel à 85°C pendant 5 minutes. Pour éliminer l'ARN, on incube le milieu réactionnel en présence de 1 µl de RNase H (Annexe B2) à 37°C pendant 20 minutes.

### 3 - Amplification par PCR :

L'exemple ici est donné spécifiquement pour le gène du 5-HT_{2C}-R. L'homme de l'art pourra aisément transposer ou adapter un tel protocole d'amplification par PCR à d'autres ARNm pouvant être édités en choisissant le couple d'amorces permettant d'amplifier spécifiquement le fragment d'ARNm comportant les sites d'édition.

Le couple d'amorces PCR9, PCR10, utilisé pour l'amplification par PCR d'un fragment du gène du 5-HT_{2C}-R a été choisi dans des régions de l'ADN identiques chez l'Homme, la souris et le rat. Les séquences du couple d'amorces sont :
PCR9 TGTCCCTAGCCATTGCTGATATGCT (SEQ ID No. 36) ; et
PCR10 GCAATCTTCATGATGGCCTTAGTCCG (SEQ ID No. 37).

Le produit d'amplification par PCR résultant a une taille de 250 paires de bases, chez les 3 espèces Homme, souris et rat.

Pour la mise en évidence de la SSCP (Single Strand Conformational Polymorphism), ces amorces peuvent être marquées par différents fluorophores tels que le C6-FAM ou HEX.

Pour l'étape d'amplification par PCR (PCR pour « Polymerase Chain Reaction »), on utilise les kit et protocole de la Platinum Taq DNA polymerase (Annexe B3). Pour l'amplification par PCR d'une région incluant le site d'édition du gène 5-HT_{2C}-R, le mélange réactionnel comprend 2µl de la solution de l'ADNc, 5 µl du tampon d'amplification (10 fois concentré ; Annexe B3), 1,5 µl d'une solution de MgCl₂ (50 mM ; Annexe B3), 1 µl d'un mélange des quatre dNTP (10 mM chacun ; Annexe B3), 1 µl de chacune des amorces PCR9 et PCR10 (solution à 10 µM), 0,5 µl de l'enzyme Platinum Taq (5 unités par µl ; Annexe B3), complété à 50 µl avec de l'eau distillée et stérile. Ce volume réactionnel est initialement chauffé dans un thermocycleur de type PTC200 (Annexe A) pendant 2 minutes à 94°C. Il est ensuite soumis à 30 cycles comprenant des phases de dénaturation à 94°C pendant 30 secondes, puis d'hybridation pendant 30 secondes à 65°C diminué de 0,3°C pour chaque cycle suivant, et d'élongation à 72°C pendant 30 secondes. Une étape finale d'élongation est conduite à 72°C pendant 6 minutes.

### 4 - Purification des produits de PCR :

Pour cette étape de purification, on utilise les kit et protocole Nucleospin Extract (Annexe B4). Le volume réactionnel obtenu par PCR (50 µl) est mélangé à 200 µl de la solution NT2 (Annexe B4) et directement déposé sur la colonne de purification. Après centrifugation à 11 000 x g pendant 1 minute, les produits d'amplification par PCR fixés sur la colonne sont lavés avec 600 µl de la solution NT3 (Annexe B4) et centrifugation à 11 000 x g pendant 1 minute. Un dernier lavage est effectué avec 200 µl de la solution NT3 (Annexe B4) et la colonne est ensuite séchée par centrifugation à 11 000 x g pendant 2 minutes. L'élution de l'ADN est obtenue en incubant la colonne avec 50 µl du tampon d'élution NE (Annexe B4) à température ambiante pendant 1 minute, suivie d'une centrifugation à 11 000 x g pendant 1 minute.

### 5 - Quantification des produits de PCR par le Bioanalyseur 2100 Agilent :

Pour déterminer la concentration de l'ADN obtenu après amplification par PCR et purification sur colonne, on utilise les kit et protocole DNA 1000 Assay (Annexe B5) et le Bioanalyseur 2100 (Annexe A). La préparation du gel est réalisée en mélangeant vigoureusement une aliquote de gel (Annexe B5) avec 25 µl de colorant (Annexe B5). Cette matrice est ensuite centrifugée à 2400 x g pendant 15 minutes, puis est injectée (9 µl) dans les micro-capillaires de la "DNA chip" (Annexe B5). Pour la quantification, les échantillons d'ADN PCR (1 µl) sont déposés dans les puits correspondants, en présence d'un indicateur de taille interne (5 µl ; Annexe B5) et d'un autre marqueur de taille (1 µl ; Annexe B5). L'électrophorèse et le calcul de la concentration de l'ADN s'effectuent en lançant le programme BioSizing du Bioanalyseur 2100 (Annexe A).

### 6 - Séparation des simples brins d'ADN par électrophorèse capillaire :

L'analyse de l'ADN amplifié par PCR marqué sur chacun des 2 brins par des fluorophores de type C6-FAM et HEX (Annexe C) est effectuée par électrophorèse en capillaire sur l'ABI PRISM 3100 (Annexe A). Les échantillons d'ADN à analyser sont tout d'abord dilués dans de l'eau à une concentration de 250 pg/µl. Pour chaque échantillon, on prépare un mélange contenant 1 µl de la solution de produit de PCR purifié et dilué, 0,5 µl de standard de migration GeneScan 500 ROX, 0,5 µl d'une solution de NaOH (0,3 N) et 10,5 µl de Formamide. Ce mélange est porté à 90°C pendant 2 minutes, puis immédiatement refroidi dans la glace. L'électrophorèse en capillaire commence par une pré-migration de 3 minutes à 15 000 volts. L'injection de l'ADN est réalisée à 1000 volts pour une durée de 22 secondes. Enfin, l'électrophorèse de l'ADN est réalisée à 15 000 volts et 18°C dans un tampon TBE une fois concentré (TBE pour Tris-Borate-EDTA) contenant le gel GeneScanPolymer (5%) et du glycérol (10%). A ce stade, la lecture de la fluorescence et l'acquisition des données sont effectuées automatiquement. La visualisation et le traitement de ces données sont effectués à partir du programme d'analyse GeneScan.

Les résultats permettent notamment d'obtenir un profil d'édition dudit ARNm dans des conditions données (longueur des produits de PCR, conditions des étapes précédentes du protocole SSCP, paramétrage du traitement des données etc.). Les paramètres de ce profil (comme le temps de migration, la hauteur et la surface des pics observés sur ce profil) seront ou pourront être comparés à des profils standards comme par exemple :
- au profil caractéristique obtenu dans ces conditions données pour chacune des formes séparées éditées (ou non) dudit ARNm (cf par exemple les figures 1C à 1O montrant les profils caractéristiques obtenus pour 13 des 32 formes éditées ou non de l'ARNm du 5-HT_{2C}-R, ce profil caractéristique ayant été obtenu pour les 32 formes potentielles SEQ ID Nos. 1 à 32) ; et/ou
- à des profils correspondant à des mélanges qualitatifs et/ou quantitatifs connus de chacune de ces formes éditées ou non, obtenus dans ces conditions données ; et/ou
- à des profils d'édition connus de ce même ARNm pour des patients sains ou présentant des pathologies confirmées, ceci pour des extraits d'ARNm de tissus spécifiques (cf. par exemple les figures 1A et 1B) dans ces mêmes conditions données,
l'ensemble de ces profils étant ou pouvant être stocké en mémoire du programme d'analyse. L'homme de l'art de l'analyse et du traitement du signal saura aisément mettre en évidence sur un tel profil d'édition les analogies et/ou les différences avec les profils d'édition standards mémorisés et annotés (pourcentages et taux d'édition, pathologie induite, traitement thérapeutique adapté, etc.).

Pour chacun de ces profils « standards » stockés dans la mémoire du programme d'analyse et obtenus dans ces conditions données, le pourcentage et/ou le taux d'édition pour chacune des formes pourra être également connu.

Pour obtenir le profil caractéristique de chacune des formes éditées (ou non) dudit ARNm par SSCP dans des conditions données (étapes 1 à 6 de l'exemple 3), on pourra procéder de la manière suivante :
- clonage et vérification par séquençage de la forme éditée ou non dont on souhaite obtenir le profil caractéristique ;
- amplification par PCR de la séquence identifiée et clonée (étape 3 de l'exemple 3) ;
- mise en oeuvre des étapes 4 à 6 de l'exemple 3.

Pour connaître les pourcentages d'édition et/ou le taux d'édition de profils standards correspondant à des mélanges connus de formes éditées ou non, il suffit de procéder avant l'étape 3 du procédé ci-avant à un mélange dans les proportions souhaitées des formes d'édition comprises dans le mélange.

Pour connaître les pourcentages et/ou taux d'édition de profils standards correspondant à des profils d'édition de référence pouvant être dans la mémoire du système d'analyse, ces profils pouvant notamment correspondre à des profils d'édition obtenus pour des patients sains ou présentant une pathologie confirmée, pour un tissu particulier, avant ou après traitement thérapeutique, etc., il suffira de procéder au clonage et au séquençage d'une partie représentative ou de toutes les séquences dudit ARNm présent dans l'extrait d'ARNm et d'en déduire la proportion de chacune des formes présentes. On pourra également dans une autre méthode utiliser les biopuces décrites sur lesquelles l'ensemble des formes d'édition dudit ARNm sera déposée.

### II) ANNEXES

### A - Liste des matériels :

ABI Prism3100 (Applied Biosystems) ;
Bioanalyseur 2100 (Agilent Technologies ; N°: DE13701290) ;
Centrifugeuse Eppendorf 5415 D (N° de série : 5425 39 178) ; et
Thermocycleur PTC200 MJ Research (N° de série : ALO46013 et ENO15975).

### B - Liste des kits :

1 - NucleoSpin RNA II (Invitrogen ; Référence : 740.955.50) ;
2 - ThermoScript RT-PCR system (Invitrogen ; Référence : 11146-024) ;
3 - Platinum Taq DNA polymerase (Invitrogen ; Référence : 10966-026) ;
4 - Nucleospin Extract (Macherey-Nagel ; Référence : 740.588.50) ; et
5 - DNA 1000 Assay (Agilent Technologies ; Référence : 5065-4449).

### C - Liste des solutions :

β-mercaptoéthanol (Sigma ; Référence : M 3148) ;
amorce PCR 9 (Proligo ; marquée en 5' par le fluorophore C6-FAM et purifiée) ;
amorce PCR10 (Proligo ; marquée en 5' par le fluorophore HEX et purifiée) ; et
TBE 10 x (Invitrogen ; Référence : 15581-044).

### EXEMPLE 4 : Sélection d'un composé capable de moduler le taux d'édition du fragment comprenant la séquence SEQ ID No. 33 (5'-AUA CGU AAU CCU A-3') de l'ARNm du 5-HT_{2C}-R chez la souris

1) Mise en contact dudit composé à évaluer avec une population de cellules eucaryotes, notamment murines, exprimant le gène dudit ARNm *(in vitro, in cellulo* ou *in vivo*)
   Exemple *(in vivo)*
   - Injection chez des souris Balb C par voie intra-péritonéale du composé à tester, environ 20 mg/ Kg.

   A des temps déterminés, par exemple jusqu'à 3 jours : les souris sont tuées et un échantillon de tissu spécifique, tel que le cortex préfontal, est prélevé, congelé puis microdisséqué.
2) Mise en évidence de la modulation de l'édition dudit fragment d'ARNm dans les cellules à partir d'un extrait d'ARNm, à l'aide du couple d'amorces PCR9 (SEQ ID No. 36) et PCR10 (SEQ ID No. 37)
   Cf. Exemple 3.
3) Comparaison du taux d'édition et/ou du profil d'édition obtenu à celui obtenu pour un extrait d'ARNm issu de la même population de cellules de souris témoins n'ayant pas été mis en contact avec le composé à évaluer, et mise en évidence de la modulation ou non du taux et/ou du profil d'édition.
   Le cas échéant,
4) Comparaison du taux d'édition et/ou du profil d'édition obtenu aux profils standards obtenus dans les mêmes conditions d'analyse pour des populations de cellules identiques après traitement avec un composé connu pour moduler le taux et/ou le profil d'édition dudit ARNm et dont l'effet thérapeutique est connu, et sélection dudit composé testé comme agent potentiel pour exercer le même effet thérapeutique si la modulation observée pour le produit testé est analogue à celle observée pour le composé connu.

### EXEMPLE 5 : Composé capable de moduler le taux d'édition du fragment comprenant la séquence SEQ ID No. 33 (5'-AUA CGU AAU CCU A-3') de l'ARNm du 5-HT_{2C}-R présentant une activité thérapeutique

Il a pu être démontré qu'un composé connu pour être un anti-dépresseur actif était capable de moduler le taux d'édition du 5-HT_{2C}-R.

## Revendications

1. Méthode SSCP permettant dans des conditions données d'analyse d'obtenir le profil d'édition de l'ARNm du 5-HT_{2C}-R, à partir d'un échantillon de tissu spécifique ou à partir d'un échantillon d'une population de cellules eucaryotes, **caractérisée en ce qu'**elle comprend les étapes suivantes :
a) l'extraction des ARN totaux dudit échantillon, suivi le cas échéant d'une purification des ARNm ;
b) la transcription inverse des ARN extraits à l'étape a) et la synthèse de l'ADN double brins ;
c) l'amplification par PCR des ADN obtenus à l'étape b) à l'aide d'un couple d'amorces spécifiques dudit ARNm du 5-HT_{2C}-R pouvant être édité, ce couple d'amorces étant choisi de manière à pouvoir amplifier toutes les formes d'édition potentiellement présentes dans l'extrait d'ARN, ces amorces étant marquées par des fluorophores ;
d) le cas échéant, la purification des produits de PCR obtenus à l'étape c) ;
e) le cas échéant, la quantification des produits de PCR obtenus à l'étape d) ;
f) la dissociation des ADN double brins en ADN simple brin, notamment par chauffage suivi d'un refroidissement brutal ;
g) la séparation des ADN simple brin par électrophorèse capillaire ; et
h) l'obtention du profil d'édition par lecture de la fluorescence et, le cas échéant, acquisition des données du profil par le système d'exploitation associé au lecteur en fluorescence.

2. Méthode SSCP selon la revendication 1, **caractérisée en ce que** le couple d'amorces utilisées à l'étape c) est choisi de manière à ce que les produits PCR obtenus sont de longueur d'au moins 100 bases.

3. Méthode SSCP selon les revendications 1 et 2, **caractérisée en ce que** le couple d'amorces est le couple d'amorces suivant, de préférence marquées par des fluorophores :
PCR9 TGTCCCTAGCCATTGCTGATATGCT (SEQ ID No. 36) ; et
PCR10 GCAATCTTCATGATGGCCTTAGTCCG (SEQ ID No. 37).

4. Méthode SSCP permettant dans des conditions données d'analyse d'obtenir le profil d'édition et le taux d'édition de l'ARNm du 5-HT_{2C}-R, à partir d'un échantillon de tissu spécifique ou à partir d'un échantillon d'une population de cellules eucaryotes, **caractérisée en ce qu'**elle comprend les étapes suivantes :
a) l'obtention du profil d'édition par la méthode SSCP selon l'une des revendications 1 à 3 ;
b) la comparaison du profil obtenu à l'étape a) avec des profils standards correspondant à :
- des profils caractéristiques obtenus dans ces conditions données pour chacune des formes séparées éditée (ou non) dudit ARNm ; et/ou
- des profils caractéristiques de mélanges qualitatifs et/ou quantitatifs connus de chacune de ces formes éditées ou non, obtenus dans ces conditions données ; et/ou
- à des profils d'édition connus dans ces mêmes conditions données, de ce même ARNm pour des patients sains ou présentant des pathologies confirmées, ceci pour des extraits d'ARNm desdits tissus spécifiques, ou encore pour ladite population de cellules eucaryotes ;
c) la sélection du profil d'édition connu correspondant au profil d'édition obtenu à l'étape a) ; et
d) l'association du taux d'édition du profil sélectionné à l'étape c) au profil d'édition obtenu à l'étape a).

5. Méthode de sélection d'un composé capable de moduler le taux et/ou le profil d'édition de l'ARNm du 5-HT_{2C}-R dans un tissu spécifique ou une population de cellules eucaryotes exprimant le gène dudit ARNm, notamment de mammifère tel que l'homme ou la souris, ledit échantillon de tissu spécifique ou échantillon d'une population de cellules eucaryotes ayant été mis préalablement en contact avec le composé à évaluer, **caractérisée en ce qu'**elle comprend les étapes suivantes :
a) l'obtention du profil d'édition par la méthode SSCP selon les revendications 1 à 4 dans des conditions données d'analyse ;
b) la comparaison du profil obtenu à l'étape a) avec ;
- soit des profils standards correspondant à des profils d'édition connus de ce même ARNm, pour un même tissu spécifique ou une même population de cellules eucaryotes, ce dans les mêmes conditions d'analyse données, ou
- soit à un profil d'édition fait en parallèle et obtenu pour un même tissu spécifique témoin ou une même population de cellules eucaryotes témoins n'ayant pas été mis en contact avec le composé à évaluer ; et
c) la sélection dudit composé à évaluer si les profils d'édition comparés à l'étape c) sont significativement différents entre eux.

6. Méthode de sélection d'un composé capable de prévenir et/ou traiter chez un patient une pathologie associée, au moins en partie, à l'édition de l'ARNm du 5-HT_{2C}-R à partir d'un tissu spécifique ou d'une population de cellules eucaryotes exprimant le gène dudit ARNm, ledit échantillon de tissu spécifique ou échantillon de population de cellules eucaryotes ayant été mis préalablement en contact avec le composé à évaluer et ledit tissu spécifique ou ladite population de cellules eucaryotes présentant avant la mise en contact du composé à tester un profil d'édition dudit ARNm caractéristique de la pathologie associée et ce, dans des conditions données d'analyse, **caractérisée en ce que** ladite méthode comprend les étapes suivantes :
a) l'obtention du profil d'édition par la méthode SSCP selon les revendications 1 à 4 dans ces conditions données d'analyse ;
b) la comparaison du profil obtenu à l'étape b) avec :
α) un profil standard correspondant à un profil d'édition connu de ce même ARNm, pour un même tissu spécifique ou pour une même population de cellules, ce dans les mêmes conditions données d'analyse, ce profil d'édition étant représentatif d'un patient sain ou ne présentant pas ladite pathologie associée ; et, le cas échéant avec :
β) un profil d'édition obtenu pour un même tissu spécifique témoin ou pour une même population de cellules témoins n'ayant pas été mis en contact avec le composé à évaluer, ce dans les mêmes conditions données d'analyse ; et
c) la sélection dudit composé à évaluer si les profils d'édition comparés à l'étape b) montrent que celui obtenu à l'étape a) est significativement identique à celui de l'étape b)α), et, le cas échéant confirmé cette sélection si celui obtenu à l'étape a) est significativement différent de celui de l'étape b)β).

7. Méthode de sélection d'un composé capable de prévenir et/ou traiter chez un patient une pathologie associée, au moins en partie, à l'édition ou non de l'ARNm du 5-HT_{2C}-R avec un même mécanisme thérapeutique ou efficacité qu'un composé connu pour moduler le profil d'édition dudit ARN et connu pour prévenir et/ou traiter chez un patient la même pathologie associée, à partir d'un tissu spécifique ou d'une population de cellules eucaryotes exprimant le gène dudit ARNm, ledit échantillon de tissu spécifique ou échantillon de population de cellules eucaryotes ayant été mis préalablement en contact avec le composé à évaluer et ledit tissu spécifique ou ladite population de cellules eucaryotes présentant avant la mise en contact du composé à tester un profil d'édition dudit ARNm caractéristique de la pathologie associée et ce, dans des conditions données d'analyse, **caractérisée en ce que** ladite méthode comprend les étapes suivantes :
a) l'obtention du profil d'édition par la méthode SSCP selon les revendications 1 à 4 dans ces conditions données d'analyse ;
b) la comparaison du profil obtenu à l'étape a) avec :
α) un profil standard correspondant à un profil d'édition connu de ce même ARNm, pour un même tissu spécifique ou pour une même population de cellules ayant été mis en contact avec ledit composé connu pour moduler le profil d'édition dudit ARN dans les mêmes conditions données d'analyse et connu pour prévenir et/ou traiter chez un patient la même pathologie associée ; et, le cas échéant avec :
β) un profil d'édition obtenu pour un même tissu spécifique témoin ou pour une même population de cellules témoins n'ayant pas été mis en contact avec le composé à évaluer, ce dans les mêmes conditions données d'analyse ; et
c) la sélection dudit composé à évaluer si les profils d'édition comparés à l'étape b) montrent que celui obtenu à l'étape a) est significativement identique à celui de l'étape b)α), et, le cas échéant confirmé cette sélection si celui obtenu à l'étape b) est significativement différent de celui de l'étape b)β).

8. Méthode de diagnostic, le cas échéant prédictif, de maladie associée, au moins en partie, à l'ARNm du 5-HT_{2C}-R. à partir d'un échantillon de tissu ou de cellules prélevé chez un patient à tester, **caractérisée en ce qu'**elle comprend les étapes suivantes :
a) l'obtention du profil d'édition dudit ARNm par la méthode SSCP selon les revendications 1 à 4 dans des conditions d'analyse données ;
b) la comparaison du profil obtenu à l'étape a) avec des profils standards correspondant à des profils d'édition connus de ce même ARNm pour des patients sains ou présentant des pathologies confirmées, ceci pour des extraits d'ARNm de même tissu ou de mêmes cellules, dans ces mêmes conditions données, ou encore pour des cellules issues de lignées cellulaires ; et
c) la sélection du profil d'édition connu correspondant au profil d'édition obtenu à l'étape a) ; et
d) l'association du diagnostic attaché au profil sélectionné à l'étape c) au patient testé.

9. Méthode de sélection d'un composé capable de moduler le taux et/ou le profil d'édition de l'ARNm du 5-HT_{2C}-R selon la revendication 5, **caractérisée en ce qu'**à l'étape c) de la méthode selon la revendication 5, on sélectionne le composé si celui-ci diminue le taux d'édition d'un site d'édition dudit ARN, site d'édition qui lorsqu'il est édité modifie la séquence des acides aminés du 5-HT_{2C}-R provenant de la traduction de l'ARNm non édité.

10. Méthode de sélection d'un composé capable de moduler le taux d'édition et/ou le profil d'édition d'un ARNm codant pour le 5-HT_{2C}-R selon la revendication 5, **caractérisée en ce qu'**à l'étape c) de la méthode selon la revendication 5, on sélectionne le composé si celui-ci augmente le taux d'édition d'un site d'édition dudit ARN, site d'édition qui lorsqu'il est édité modifie la séquence des acides aminés du 5-HT_{2C}-R provenant de la traduction de l'ARNm non édité.

## Claims

1. SSCP method for obtaining, under given analytical conditions, the editing profile of 5-HT_{2C}-R mRNA, using a specific tissue sample or using a sample of a population of eukaryotic cells, **characterised in that** it comprises the following steps:
a) extraction of the total RNA of said sample, followed, where appropriate, by purification of the mRNA;
b) reverse transcription of the RNA extracted in step a) and synthesis of the double-stranded DNA;
c) PCR amplification of the DNA obtained in step b) using a pair of primers specific for said 5-HT_{2C}-R mRNA, this pair of primers being chosen so as to be able to amplify all the editing forms potentially present in the RNA extract, these primers being labelled with fluorophores;
d) where appropriate, purification of the PCR products obtained in step c);
e) where appropriate, quantification of the PCR products obtained in step d);
f) dissociation of the double-stranded DNAs to single-stranded DNAs, in particular by heating followed by abrupt cooling;
g) separation of the single-stranded DNAs by capillary electrophoresis; and
h) obtaining of the editing profile by reading of the fluorescence and, where appropriate, acquisition of the profile data by means of the operating system associated with the fluorescence reader.

2. SSCP method according to claim 1, **characterised in that** the pair of primers used in step c) is chosen such that the PCR products obtained are at least 100 bases in length.

3. SSCP method according to claims 1 and 2, **characterised in that** the pair of primers is the following pair of primers, preferably labelled with fluorophores:
PCR9 TGTCCCTAGCCATTGCTGATATGCT (SEQ ID No. 36);
and
PCR10 GCAATCTTCATGATGGCCTTAGTCCG (SEQ ID No. 37).

4. SSCP method for obtaining, under given analytical conditions, the editing profile and the editing rate of 5-HT_{2C}-R mRNA, using a specific tissue sample or using a sample of a population of eukaryotic cells, **characterised in that** it comprises the following steps:
a) obtaining the editing profile by means of the SSCP method according to any of claims 1 to 3;
b) comparing the profile obtained in step a) with standard profiles corresponding to:
- characteristic profiles obtained, under these given conditions, for each of the edited (or unedited) separate forms of said mRNA; and/or
- characteristic profiles of known qualitative and/or quantitative mixtures of each of these edited or unedited forms, obtained under these given conditions; and/or
- known editing profiles, under these same given conditions, of this same mRNA for normal patients or patients presenting confirmed pathologies, for mRNA extracts of said specific tissues, or else for said population of eukaryotic cells;
c) selecting the known editing profile corresponding to the editing profile obtained in step a); and
d) associating the editing rate of the profile selected in step c) with the editing profile obtained in step a).

5. Method for selecting a compound capable of modulating the editing rate and/or the editing profile of 5-HT_{2C}-R mRNA in a specific tissue or a population of eukaryotic cells expressing the gene of said mRNA, in particular from a mammal, such as human or mouse, said specific tissue sample or sample of a population of eukaryotic cells having been previously brought into contact with the compound to be evaluated, **characterised in that** it comprises the following steps:
a) obtaining the editing profile by means of the SSCP method according to claims 1 to 4, under given analytical conditions;
b) comparing the profile obtained in step a) with:
- either standard profiles corresponding to known editing profiles of this same mRNA, for the same specific tissue or the same population of eukaryotic cells, under the same given analytical conditions,
- or an editing profile determined in parallel and obtained for the same control specific tissue or the same population of control eukaryotic cells that have not been brought into contact with the compound to be evaluated; and
c) selecting said compound to be evaluated if the editing profiles compared in step c) are significantly different from one another.

6. Method for selecting a compound capable of preventing and/or treating, in a patient, a pathology associated, at least in part, with the editing of 5-HT_{2C}-R mRNA, using a specific tissue or a population of eukaryotic cells expressing the gene of said mRNA, said specific tissue sample or sample of a population of eukaryotic cells having been previously brought into contact with the compound to be evaluated and said specific tissue or said population of eukaryotic cells exhibiting, before being brought into contact with the compound to be tested, an editing profile of said mRNA characteristic of the associated pathology, under given analytical conditions, **characterised in that** said method comprises the following steps:
a) obtaining the editing profile by means of the SSCP method according to claims 1 to 4, under these given analytical conditions;
b) comparing the profile obtained in step b) with:
α) a standard profile corresponding to a known editing profile of this same mRNA, for the same specific tissue or for the same population of cells, under the same given analytical conditions, this editing profile being representative of a normal patient or a patient not presenting said associated pathology; and, where appropriate, with:
β) an editing profile obtained for the same control specific tissue or for the same population of control cells that have not been brought into contact with the compound to be evaluated, under the same given analytical conditions; and
c) selecting said compound to be evaluated if the editing profiles compared in step b) show that the one obtained in step a) is significantly identical to the one in step b)α), and, where appropriate, confirming this selection if the profile obtained in step a) is significantly different from the one in step b)β).

7. Method for selecting a compound capable of preventing and/or treating, in a patient, a pathology associated, at least in part, with the editing or non-editing of 5-HT_{2C}-R mRNA, with the same therapeutic mechanism or effectiveness as a compound known to modulate the editing profile of said RNA and known to prevent and/or treat, in a patient, the same associated pathology, using a specific tissue or a population of eukaryotic cells expressing the gene of said mRNA, said specific tissue sample or sample of a population of eukaryotic cells having been previously brought into contact with the compound to be evaluated and said specific tissue or said population of eukaryotic cells exhibiting, before being brought into contact with the compound to be tested, an editing profile of said mRNA characteristic of the associated pathology, under given analytical conditions, **characterised in that** said method comprises the following steps:
a) obtaining the editing profile by means of the SSCP method according to claims 1 to 4, under these given analytical conditions;
b) comparing the profile obtained in step a) with:
α) a standard profile corresponding to a known editing profile of this same mRNA, for the same specific tissue or for the same population of cells having been brought into contact with said compound known to modulate the editing profile of said RNA, under the same given analytical conditions, and known to prevent and/or treat, in a patient, the same associated pathology; and, where appropriate, with:
β) an editing profile obtained for the same control specific tissue or for the same population of control cells that have not been brought into contact with the compound to be evaluated, under the same given analytical conditions; and
c) selecting said compound to be evaluated if the editing profiles compared in step b) show that the one obtained in step a) is significantly identical to the one in step b)α), and, where appropriate, confirming this selection if the profile obtained in step b) is significantly different from the one in step b)β).

8. Method for diagnosing, where appropriate for predicting, a disease associated, at least in part, with 5-HT_{2C}-R mRNA, using a tissue or cell sample taken from a patient to be tested, **characterised in that** it comprises the following steps:
a) obtaining the editing profile of said mRNA by means of the SSCP method according to claims 1 to 4, under given analytical conditions;
b) comparing the profile obtained in step a) with standard profiles corresponding to known editing profiles of this same mRNA for normal patients or patients exhibiting confirmed pathologies, for mRNA extracts of the same tissue or of the same cells, under these same given conditions, or else for cells derived from cell lines; and
c) selecting the known editing profile corresponding to the editing profile obtained in step a); and
d) associating the diagnosis related to the profile selected in step c), with the patient tested.

9. Method for selecting a compound capable of modulating the editing rate and/or the editing profile of 5-HT_{2C}-R mRNA, according to claim 5, **characterised in that**, in step c) of the method according to claim 5, the compound is selected if it decreases the editing rate of an editing site of said RNA, the editing site, when it is edited, modifying the amino acid sequence of 5-HT_{2C}-R originating from the translation of the unedited mRNA.

10. Method for selecting a compound capable of modulating the editing rate and/or the editing profile of mRNA encoding 5-HT_{2C}-R, according to claim 5, **characterised in that**, in step c) of the method according to claim 5, the compound is selected if it increases the editing rate of an editing site of said RNA, the editing site, when it is edited, modifying the amino acid sequence of 5-HT_{2C}-R originating from the translation of the unedited mRNA.

## Patentansprüche

1. SSCP-Verfahren, mit dem unter bestimmten Analysebedingungen das Editierungsprofil der mRNA von 5-HT_{2C}-R erhalten werden kann, aus einer Probe eines spezifischen Gewebes oder aus einer Probe einer Population von Eukaryotenzellen, **dadurch gekennzeichnet, dass** es folgende Schritte umfasst:
a) Extraktion der Gesamt-RNA aus der Probe, gegebenenfalls gefolgt von einer Reinigung der mRNA;
b) inverse Transkription der RNA, die in Schritt a) extrahiert wurde, und Synthese der doppelsträngigen DNA;
c) mittels PCR Vervielfältigung der DNA, die in Schritt b) erhalten wurde, mithilfe eines Paars spezifischer Primer für die mRNA von 5-HT_{2C}-R, die editiert werden kann, wobei das Paar von Primern derart gewählt wird, dass sämtliche Editierungsformen, die potenziell in dem RNA-Extrakt vorhanden sind, vervielfältigt werden können, wobei diese Primer mit Fluorophoren markiert sind;
d) gegebenenfalls Reinigung der PCR-Produkte, die in Schritt c) erhalten wurden;
e) gegebenenfalls Quantifizierung der PCR-Produkte, die in Schritt d) erhalten wurden;
f) Trennung der doppelsträngigen DNA in einsträngige DNA, insbesondere durch Erwärmen mit anschließender plötzlicher Abkühlung;
g) Trennung der einsträngigen DNA durch Kapillarelektrophorese, und
h) Erhalten des Editierungsprofils durch Ablesen der Fluoreszenz und gegebenenfalls Erfassen der Daten des Profils mit dem Auswertungssystem, das dem Fluoreszenz-Reader zugehörig ist.

2. SSCP-Verfahren nach Anspruch 1, **dadurch gekennzeichnet, dass** das Paar von Primern, das in Schritt c) verwendet wird, so gewählt wird, dass die gewonnenen PCR-Produkte eine Länge von mindestens 100 Basen aufweisen.

3. SSCP-Verfahren nach Anspruch 1 und 2, **dadurch gekennzeichnet, dass** das Paar von Primern das folgende Paar von Primern ist, die vorzugsweise mit Fluorophoren markiert sind:
PCR9 TGTCCCTAGCCATTGCTGATATGCT (Sequenz-Nr. 36); und
PCR10 GCAATCTTCATGATGGCCTTAGTCCG (Sequenz-Nr. 37).

4. SSCP-Verfahren, mit dem unter bestimmten Analysebedingungen das Editierungsprofil und die Editierungsrate der mRNA von 5-HT_{2C}-R erhalten werden kann, aus einer Probe eines spezifischen Gewebes oder aus einer Probe einer Population von Eukaryotenzellen, **dadurch gekennzeichnet, dass** es folgende Schritte umfasst:
a) Erhalten des Editierungsprofils mit dem SSCP-Verfahren nach einem der Ansprüche 1 bis 3;
b) Vergleichen des Profils, das in Schritt a) erhalten wurde, mit Standardprofilen, die Folgendem entsprechen:
- charakteristischen Profilen, die unter diesen bestimmten Bedingungen für jede der getrennten editierten (oder nicht editierten) Formen der mRNA erhalten werden;
und/oder
- charakteristischen Profilen bekannter qualitativer und/oder quantitativer Mischungen aus jeder dieser editierten oder nicht editierten Formen, die unter diesen bestimmten Bedingungen erhalten werden;
und/oder
- bekannten Editierungsprofilen unter denselben bestimmten Bedingungen von derselben mRNA für gesunde Patienten oder Patienten mit bestätigten Erkrankungen, dies für mRNA-Extrakte der spezifischen Gewebe oder auch für die Population von Eukaryotenzellen;
c) Auswählen des bekannten Editierungsprofils, das dem Editierungsprofil entspricht, das in Schritt a) erhalten wurde; und
d) Verbinden der Editierungsrate des Profils, das in Schritt c) ausgewählt wurde, mit dem Editierungsprofil, das in Schritt a) erhalten wurde.

5. Verfahren zum Auswählen einer Verbindung, die die Editierungsrate und/oder das Editierungsprofil der mRNA von 5-HT_{2C}-R in einem spezifischen Gewebe oder einer Population von Eukaryotenzellen abwandeln kann, das bzw. die das Gen der mRNA exprimiert, insbesondere von einem Säugetier wie Mensch oder Maus, wobei die Probe eines spezifischen Gewebes oder die Probe einer Population von Eukaryotenzellen vorher mit der zu beurteilenden Verbindung in Kontakt gebracht wurde, **dadurch gekennzeichnet, dass** es folgende Schritte umfasst:
a) Erhalten des Editierungsprofils mit dem SSCP-Verfahren nach den Ansprüchen 1 bis 4 unter bestimmten Analysebedingungen;
b) Vergleichen des Profils, das in Schritt a) erhalten wurde, mit
- entweder Standardprofilen, die bekannten Editierungsprofilen derselben mRNA entsprechen, für dasselbe spezifische Gewebe oder dieselbe Population von Eukaryotenzellen, dies unter denselben bestimmten Analysebedingungen, oder
- einem Editierungsprofil, das parallel bestimmt wird und für dasselbe spezifische Kontrollgewebe oder dieselbe Population von Kontroll-Eukaryotenzellen erhalten wird, das bzw. die nicht mit der zu beurteilenden Verbindung in Kontakt gebracht wurde; und
c) Auswählen der zu beurteilenden Verbindung, wenn die Editierungsprofile, die in Schritt c) verglichen wurden, wesentliche Unterschiede zueinander aufweisen.

6. Verfahren zum Auswählen einer Verbindung, die bei einem Patienten eine Erkrankung verhüten und/oder behandeln kann, die zumindest teilweise mit der Editierung der mRNA von 5-HT_{2C}-R in Zusammenhang steht, auf der Basis eines spezifischen Gewebes oder einer Population von Eukaryotenzellen, wobei die Probe eines spezifischen Gewebes oder die Probe einer Population von Eukaryotenzellen vorher mit der zu beurteilenden Verbindung in Kontakt gebracht wurde, und das spezifische Gewebe oder die Population der Eukaryotenzellen vor dem Kontaktieren mit der zu prüfenden Verbindung ein Editierungsprofil der mRNA aufweist, das für die zugehörige Erkrankung charakteristisch ist, und dies unter bestimmten Analysebedingungen, **dadurch gekennzeichnet, dass** das Verfahren folgende Schritte umfasst:
a) Erhalten des Editierungsprofils mit dem SSCP-Verfahren nach den Ansprüchen 1 bis 4 unter diesen bestimmten Analysebedingungen;
b) Vergleichen des Profils, das in Schritt b) erhalten wurde, mit:
α) einem Standardprofil, das einem bekannten Editierungsprofil derselben mRNA entspricht, für dasselbe spezifische Gewebe oder für dieselbe Population von Zellen, dies unter denselben bestimmten Analysebedingungen, wobei dieses Editierungsprofil typisch ist für einen gesunden Patienten oder einen Patienten, der die zugehörige Erkrankung nicht hat; und gegebenenfalls mit:
β) einem Editierungsprofil, das für dasselbe spezifische Kontrollgewebe oder für dieselbe Population von Kontrollzellen erhalten wird, das bzw. die nicht mit der zu beurteilenden Verbindung in Kontakt gebracht wurde, dies unter denselben bestimmten Analysebedingungen, und
c) Auswählen der zu beurteilenden Verbindung, wenn die Editierungsprofile, die in Schritt b) verglichen wurden, zeigen, dass das Profil, das in Schritt a) erhalten wurde, signifikant identisch zu dem aus Schritt b)α) ist, und gegebenenfalls Bestätigen dieser Auswahl, wenn das Profil, das in Schritt a) erhalten wurde, wesentliche Unterschiede zu dem aus Schritt b)β) aufweist.

7. Verfahren zum Auswählen einer Verbindung, die bei einem Patienten eine Erkrankung verhüten und/oder behandeln kann, die zumindest teilweise mit der Editierung oder Nichteditierung der mRNA von 5-HT_{2C}-R in Zusammenhang steht, mit demselben therapeutischen Mechanismus oder derselben Wirksamkeit wie eine Verbindung, die bekanntermaßen das Editierungsprofil der RNA abwandelt und bekanntermaßen bei einem Patienten dieselbe zugehörige Erkrankung verhütet und/oder behandelt, auf der Basis eines spezifischen Gewebes oder einer Population von Eukaryotenzellen, das bzw. die das Gen der mRNA exprimiert, wobei die Probe eines spezifischen Gewebes oder die Probe einer Population von Eukaryotenzellen vorher mit der zu beurteilenden Verbindung in Kontakt gebracht wurde, und das spezifische Gewebe oder die Population der Eukaryotenzellen vor dem Kontaktieren mit der zu prüfenden Verbindung ein Editierungsprofil der mRNA aufweist, das für die zugehörige Erkrankung charakteristisch ist, und dies unter bestimmten Analysebedingungen, **dadurch gekennzeichnet, dass** das Verfahren folgende Schritte umfasst:
a) Erhalten des Editierungsprofils mit dem SSCP-Verfahren nach den Ansprüchen 1 bis 4 unter diesen bestimmten Analysebedingungen;
b) Vergleichen des Profils, das in Schritt a) erhalten wurde, mit:
α) einem Standardprofil, das einem bekannten Editierungsprofil derselben mRNA entspricht, für dasselbe spezifische Gewebe oder dieselbe Population von Zellen, das bzw. die mit der Verbindung in Kontakt gebracht wurde, die bekanntermaßen das Editierungsprofil der RNA abwandelt, unter denselben bestimmten Analysebedingungen, und bei einem Patienten bekanntermaßen dieselbe zugehörige Erkrankung verhütet und/oder behandelt; und gegebenenfalls mit:
β) einem Editierungsprofil, das für dasselbe spezifische Kontrollgewebe oder für dieselbe Population von Kontrollzellen erhalten wird, das bzw. die nicht mit der zu beurteilenden Verbindung in Kontakt gebracht wurde, dies unter denselben bestimmten Analysebedingungen, und
c) Auswählen der zu beurteilenden Verbindung, wenn die Editierungsprofile, die in Schritt b) verglichen wurden, zeigen, dass das Profil, das in Schritt a) erhalten wurde, signifikant identisch zu dem aus Schritt b)α) ist, und gegebenenfalls Bestätigen dieser Auswahl, wenn das Profil, das in Schritt b) erhalten wurde, wesentliche Unterschiede zu dem aus Schritt b)β) aufweist.

8. Diagnoseverfahren, gegebenenfalls Vorhersageverfahren, für eine Krankheit, die zumindest teilweise mit der mRNA von 5-HT_{2C}-R zusammenhängt, auf der Basis einer Gewebeprobe oder Zellprobe, die einem zu untersuchenden Patienten entnommen wird, **dadurch gekennzeichnet, dass** es folgende Schritte umfasst:
a) Erhalten des Editierungsprofils der mRNA mit dem SSCP-Verfahren nach den Ansprüchen 1 bis 4 unter bestimmten Analysebedingungen;
b) Vergleichen des Profils, das in Schritt a) erhalten wurde, mit Standardprofilen, die bekannten Editierungsprofilen derselben mRNA bei gesunden Patienten oder Patienten mit bestätigten Erkrankungen entsprechen, dies für mRNA-Extrakte desselben Gewebes oder derselben Zellen, unter denselben bestimmten Bedingungen, oder auch für Zellen, die aus Zelllinien stammen; und
c) Auswählen des bekannten Editierungsprofils, das dem Editierungsprofil entspricht, das in Schritt a) erhalten wurde; und
d) Verknüpfen der Diagnose, die mit dem Profil zusammenhängt, das in Schritt c) ausgewählt wurde, mit dem untersuchten Patienten.

9. Verfahren zum Auswählen einer Verbindung, die die Editierungsrate und/oder das Editierungsprofil der mRNA von 5-HT_{2C}-R abwandeln kann, nach Anspruch 5, **dadurch gekennzeichnet, dass** in Schritt c) des Verfahrens nach Anspruch 5 die Verbindung ausgewählt wird, wenn sie die Editierungsrate einer Editierungsstelle der RNA verringert, wobei die Editierungsstelle, wenn sie editiert ist, die Aminosäurensequenz von 5-HT_{2C}-R, der bei der Translation der nicht editierten mRNA entsteht, ändert.

10. Verfahren zum Auswählen einer Verbindung, die die Editierungsrate und/oder das Editierungsprofil einer mRNA abwandeln kann, die für 5-HT_{2C}-R kodiert, nach Anspruch 5, **dadurch gekennzeichnet, dass** in Schritt c) des Verfahrens nach Anspruch 5 die Verbindung ausgewählt wird, wenn sie die Editierungsrate einer Editierungsstelle der RNA erhöht, wobei die Editierungsstelle, wenn sie editiert ist, die Aminosäurensequenz von 5-HT_{2C}-R, der bei der Translation der nicht editierten mRNA entsteht, ändert.
